# FASCICULE DE BREVET EUROPEEN

(11) **EP 1 436 304 B1**
(45) Date de publication et mention de la délivrance du brevet: **09.03.2005**
(21) Numéro de dépôt: 02800152.7
(22) Date de dépôt: 16.09.2002
(51) Int. Cl.: C07F 9/655, C07F 15/00, B01J 31/24, C07B 53/00

(54) **DIPHOSPHINES ET LEUR UTILISATION EN SYNTHESE ASYMETRIQUE**
NEUE DIPHOSPHINE, DEREN KOMPLEXE MIT ÜBERGANGSMETALLEN UND DEREN VERWENDUNG IN DER ASYMMETRISCHEN SYNTHESE
NOVEL DISPHOSPINES, THEIR COMPLEXES WITH TRANSITION METALS AND THEIR USE IN ASYMMETRIC SYNTHESIS

(30) Priorité: 28.09.2001 FR 0112499
(43) Date de publication de la demande: 14.07.2004
(73) Titulaire: Synkem, 21300 Chenove (FR)
(72) Inventeur: DUPRAT DE PAULE, Sébastien, F-75020 Paris (FR); CHAMPION, Nicolas, F-21000 Dijon (FR); VIDAL, Virginie, F-75013 Paris (FR); GENET, Jean-Pierre, F-91370 Verrieres-Le-Buisson (FR); DELLIS, Philippe, F-21000 Dijon (FR)
(74) Mandataire: Nevant, Marc
(86) Numéro de dépôt international: PCT/FR2002/003146
(87) Numéro de publication internationale: WO 2003/029259

(56) Documents cités:
- EP-A- 0 850 945
- TSIVUNIN V.S. ET AL.: "Phosphorylation of 1,4-benzodioxane" JOURNAL OF GENERAL CHEMISTRY USSR., vol. 51, no. 7, - 20 décembre 1981 (1981-12-20) pages 1317-1319, XP002199773 CONSULTANTS BUREAU. NEW YORK., US
- PAI C-C ET AL: "Synthesis of new chiral diphosphine ligand (BisbenzodioxanPhos) and its application in asymmetric catalytic hydrogenation" TETRAHEDRON LETTERS, ELSEVIER SCIENCE PUBLISHERS, AMSTERDAM, NL, vol. 43, no. 15, 8 avril 2002 (2002-04-08), pages 2789-2792, XP004345895 ISSN: 0040-4039

## Description

La présente invention concerne de nouvelles diphosphines racémiques ou chirales, utiles comme ligands bi-dentés dans la synthèse de complexes métalliques, et plus particulièrement de catalyseurs destinés notamment à l'hydrogénation asymétrique catalytique.

La catalyse asymétrique présente l'avantage de conduire directement à la préparation d'isomères optiquement purs, par induction asymétrique, sans qu'il soit nécessaire de procéder au dédoublement des mélanges racémiques. L'art antérieur décrit déjà certains ligands di-phosphorés, utilisés couramment dans la préparation de complexes métalliques, pour la catalyse asymétrique des réactions d'hydrogénation, de carbonylation, d'hydrosilylation, de formation de liaisons C-C ou même d'isomérisation asymétrique d'allylamines. On pourra notamment citer le 2,2'-bis(diphénylphosphino)-1,1'-binaphtyle (BINAP) ou le [(5,6),(5',6')-bis(méthylènedioxy)biphényl-2,2'-diyl]bis(diphénylphosphine) (SEGPHOS) décrit dans la demande de brevet publiée sous le numéro EP 850 945.

La mise au point de nouveaux ligands chiraux est souhaitable, de façon à améliorer la sélectivité des réactions (diastéréosélectivité et énantiosélectivité).

La présente invention a donc pour objet de nouveaux dérivés de diphosphine, sous forme racémique ou optiquement pure, de formule (I) : 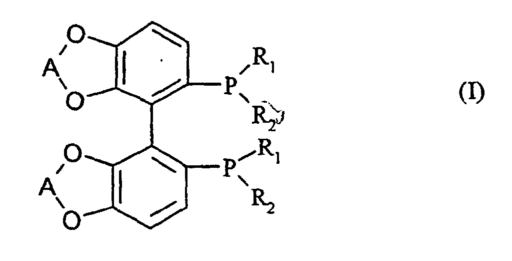 dans laquelle :
- R₁ et R₂ représentent chacun indépendamment :
   un groupe (C₅-C₇)cycloalkyle, un groupe phényle éventuellement substitué par un ou plusieurs groupes (C₁-C₄)alkyle, (C₁-C₄)alcoxy, di(C₁-C₄)alkylamino, ou par un atome d'halogène ou,
   un groupe hétéroaryle à 5 chaînons ;
- A représente un groupe éthylène (CH₂-CH₂) ou un groupe CF₂.

Par groupe hétéroaryle à 5 chaînons, on entend par exemple un groupe 2-furanyle, 3-furanyle, 2-benzofuranyle ou 3-benzofuranyle.

Par alkyle, on entend un groupe hydrocarboné, saturé, linéaire ou ramifié.

Par (C₁-C₄)alkyle, on entend un groupe alkyle comprenant de 1 à 4 atomes de carbone.

Le terme alcoxy désigne le radical O-alkyle où alkyle est tel que défini ci-dessus.

Par atome d'halogène, on entend un atome de chlore, brome, fluor ou iode.

Selon un aspect particulier, la présente invention concerne les composés de formule (1) dans laquelle R₁ et R₂ sont identiques et plus particulièrement dans laquelle R₁ et R₂ sont identiques et représentent un groupe phényle.

Les composés de formule (I) selon la présente invention peuvent être préparés selon le procédé présenté dans le SCHEMA 1 ci-après dans lequel R₁, R₂ et A sont tels que définis pour (I) et R représente un groupe (C₁-C₄)alkyle ou un groupe phényle éventuellement substitué.

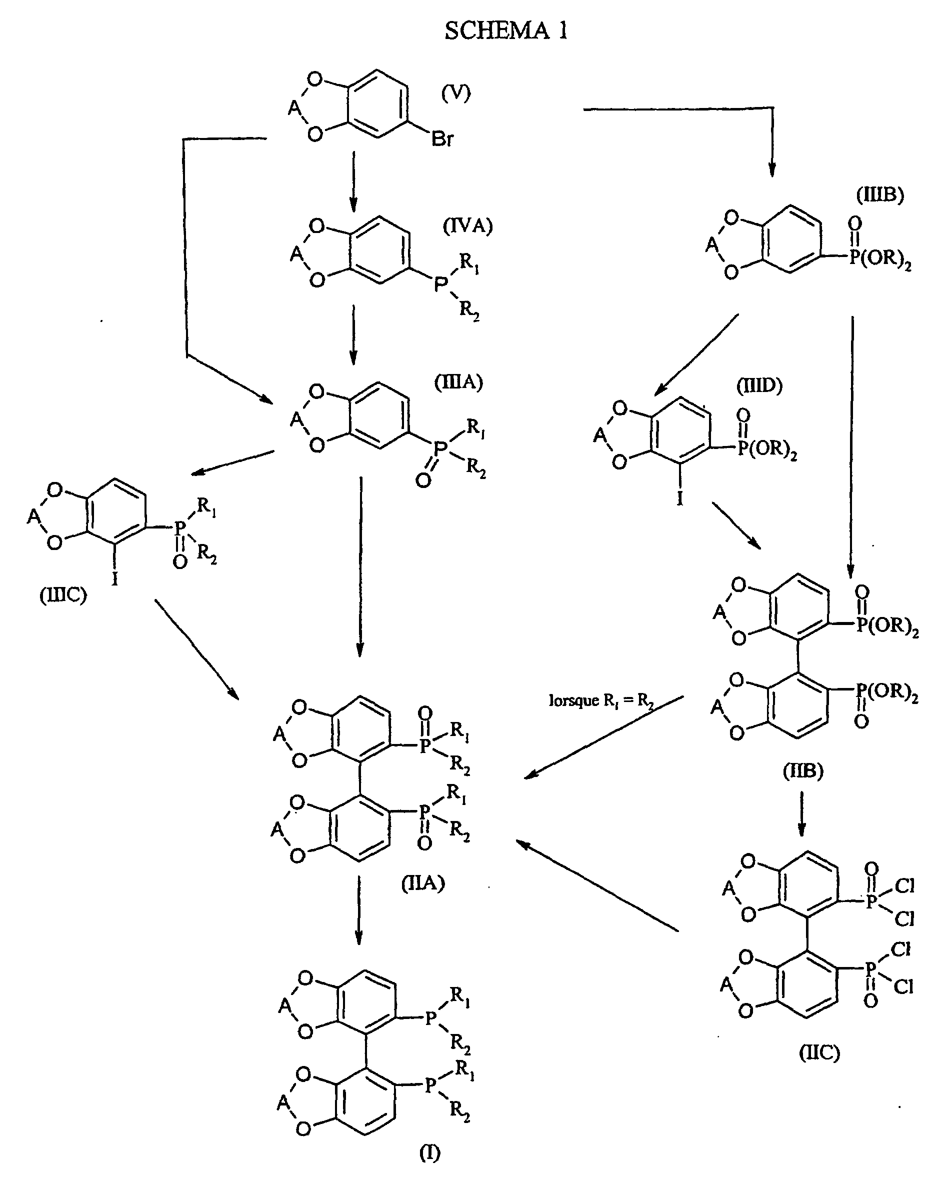

Les composés de formule (I), sous forme optiquement pure, (R) ou (S) ou sous forme racémique, peuvent être préparés par réduction du composé de formule (VA) ; 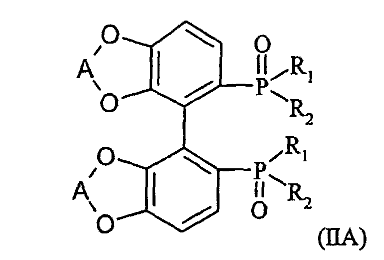 dans laquelle R₁ , R₂ et A sont tels que définis pour (I), respectivement sous la forme optiquement pure correspondante (R) ou (S), ou sous la forme racémique ; par exemple, par action d'un agent réducteur tel que le trichlorosilane, en présence d'une amine comme la tributylamine.

Le composé (IIA) sous forme optiquement pure est, par exemple, obtenu par dédoublement du composé (IIA) sous forme racémique, par formation d'un complexe avec l'acide (-)-L-dibenzoyltartrique ou l'acide (+)-D-dibenzoyltartrique mais aussi grâce à d'autres acides chiraux comme décrit dans l'art antérieur pour ce type de dédoublement.

Les énantiomères peuvent être également préparés par séparation par chromatographie sur phase chirale.

Les composés de formule (IIA), sous forme racémique ou optiquement pure, intermédiaires dans la synthèse des composés de formule (I), sont des composés nouveaux et font partie intégrante de l'invention.

Les composés de formule (IIA) peuvent être préparés à partir des composés (IIIA) : 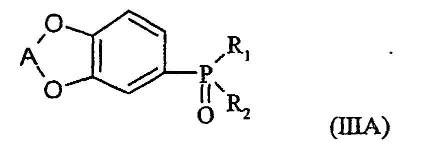 dans laquelle R₁, R₂ et A sont tels que définis pour (I), par action d'un organolithien, tel que le *tert*-butyllithium, en présence de trichlorure de fer ou d'un autre oxydant adéquat.

Les composés (IIA) peuvent être également préparés à partir du dérivé (IIIA) en deux étapes : iodation du composé (IIIA) pour obtenir un dérivé iodé de formule (IIIC), suivie d'une réaction de couplage de type Ullman à l'aide de cuivre.

Les composés (IIA) dans lesquels R₁ et R₂ sont identiques peuvent être préparés d'une troisième façon, à partir des composés de formule (IIB) dans laquelle R représente un groupe (C₁-C₄)alkyle ou un groupe phényle substitué ou non. Dans ce troisième procédé, les composés (IIB) sont mis en présence d'un organométallique de formule R₁Li ou R₁MgX dans laquelle R₁ est tel que défini pour (I) et X représente un atome d'halogène.

Les composés (IIB) peuvent exister sous forme racémique ou chirale et peuvent être dédoublés comme les composés de formule (IIA) par des acides chiraux ou par chromatographie sur phase chirale.

Les composés de formule (IIB) sont nouveaux et font partie intégrante de l'invention.

Les composés (IIA), dans lesquels R₁ et R₂ sont identiques peuvent aussi être préparés à partir des composés de formule IIB, selon un procédé en 2 étapes consistant à, dans un premier temps faire agir le chlorure de thionyle, en présence d'un solvant, pour obtenir le dérivé halogéné (IIC) 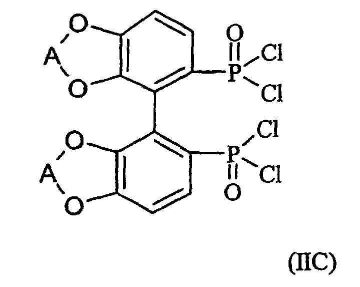 et dans un second temps, faire agir sur ce composé IIc un organométallique, notamment un organolithien de formule R₁Li ou un organomagnésien de formule R₁MgX, dans lesquelles R₁ est tel que défini pour (I) et X représente un atome d'halogène, pour obtenir le composé de formule IIA attendu.

Le composé de formule (IIIA) peut être préparé par oxydation de la phosphine de formule (IVA) : 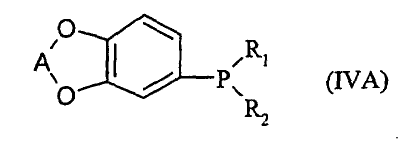 dans laquelle R₁, R₂ et A sont tels que définis pour (I), par action d'une solution d'eau oxygénée dans le méthanol ou à l'aide d'autres réactifs d'oxydation des phosphines bien connus de l'homme de l'art.

La phosphine (IVA) peut elle-même être préparée à partir du composé bromé correspondant (V), par action d'un organolithien tel que le *n*-butyllithium, puis de la phosphine R₁R₂PCl avec R₁ et R₂ tels que définis pour (I), à une température proche de - 70°C.

Le composé (IIIA) peut également être préparé directement à partir du composé (V), par action de magnésium dans le tétrahydrofurane pour former un réactif de Grignard, suivie de la réaction avec le chlorure de phosphinyle R₁R₂P(O)Cl, avec R₁ et R₂ tels que définis pour (I).

Le composé (IIIA) peut également être préparé à partir du composé (V) via le composé (IVA), par action de magnésium dans le tétrahydrofurane pour former un réactif de Grignard, suivie de la réaction avec la phosphine R₁R₂PCl, avec R₁ et R₂ tels que définis pour (I) puis d'une oxydation par action d'une solution d'eau oxygénée dans le méthanol ou à l'aide d'autres réactifs d'oxydation des phosphines bien connus de l'homme de l'art.

Les composés de formule (IIIA), (IIIC) et (IVA) sont nouveaux et font partie intégrante de l'invention.

Les dérivés de formule (IIB) peuvent être obtenus en une étape à partir du dérivé (IIIB) dans lequel R est défini comme pour le dérivé (IIB) par action d'un organolithien tel que le sec-butyllithium en présence de trichlorure de fer ou d'un autre oxydant adéquat.

Les dérivés de formule (IIB) peuvent être également obtenus en deux étapes à partir des dérivés de formule (IIIB) : par iodation, on obtient les dérivés iodés (IIID) qui sont soumis à l'action du cuivre dans une réaction de type Ullman pour obtenir les dérivés (IIB).

Les composés de formule (IIIB) peuvent être obtenus à partir des composés de formule (V) par réaction avec le magnésium dans un éther pour former un organomagnésien et réaction de ce dernier avec un dérivé Cl-P(O)(OR)₂ dans lequel R est tel que défini dans (IIB).

Les composés de formule (IIIB) peuvent également être obtenus à partir des composés de formule (V) par réaction avec le chlorure de nickel à une température de l'ordre de 100 à 160 °C en présence d'un dérivé P(OR)₃ dans lequel R est défini tel que pour (IIB).

Les composés (IIIB) dans lesquels A représente CF₂ sont nouveaux et font partie intégrante de l'invention. Les composés (IIIB) pour lesquels A représente l'éthylène et R représente un groupe phényle, méthyle ou (C₃-C₄)alkyle sont nouveaux et font partie intégrante de l'invention.

Un aspect ultérieur de l'invention concerne l'utilisation d'un composé de formule (I), comme ligand pour la préparation d'un complexe métallique, utile comme catalyseur chiral dans la catalyse asymétrique.

La présente invention a également pour objet les catalyseurs métalliques chiraux comprenant au moins un ligand de formule (I) sous forme racémique ou de préférence optiquement pure. Dans le cas où le ligand de formule (I) est sous forme racémique, la chiralité du complexe métallique est obtenue par l'intermédiaire d'un autre ligand chiral, par exemple de type diamine chirale.

Les catalyseurs métalliques selon la présente invention pourront être utilisés pour la catalyse asymétrique des réactions d'hydrogénation, d'hydroboration de composés insaturés, d'isomérisation d'oléfines, d'alkylation allylique et, de façon générale, des réactions de formation de liaisons C-C (telles que les additions-1,4 d'acides boroniques), de cyclisation asymétrique de 4-penténals (J. Org. Chem. 2000, 65, 5806-16), de cyclisation ène-yne (Angew. Chem., Int. Ed. 2001, 40(1), 249-53), de substitution allylique d'enolates (Angew. Chem., Int. Ed. 2000, 39(19), 3494-7), de formation d'acides α-aminés aromatiques (Angew. Chem., Int. Ed. 2000, 39(22), 4114-6).

Selon un mode de réalisation préféré de l'invention, les catalyseurs métalliques sont utilisés pour l'hydrogénation des liaisons C=O, C=C et C=N. Les catalyseurs utilisables dans ce type de réaction sont de préférence des catalyseurs au rhodium, au ruthénium, au palladium, à l'iridium, au nickel ou au cuivre.

Selon un mode particulier, l'invention concerne les catalyseurs métalliques chiraux de formule (VI) :

MₘLₙXₚS_{q} (VI)

dans laquelle :
- M représente un métal choisi parmi le rhodium, le ruthénium, l'iridium, le palladium, le nickel ou le cuivre ;
- L représente un composé (I) chiral ;
- X, S, m, n, p et q sont définis comme suit :
   - lorsque M = Rh, alors X = Cl, Br ou I ; m = n = p = 2; q = 0;
   - lorsque M = Ru, alors : X = -OC(O)CH₃ ; m = n = 1 ; p = 2 ; q = 0;
      ou bien X = Br ; m = n = 1 ; p = 2 ; q = 0;
      ou bien X = Cl ; S = N(CH₂CH₃)₃ ; m = n = 2 ; p = 4 ; q = 1 ;
      ou bien X=méthylallyl ; m=n= 1 ; p = 2 ; q = 0;
      ou bien X = Cl ; S = pyridine ; m = n = 1 ; p = q = 2 ;
      ou bien X= Cl, S = 1,2-diamine chirale ; m = n = 1 ; p = q = 2 ou p = 2 , q = 1 ;
   - lorsque M = Ir, alors X = Cl, Br ou I ; m = n = p = 2 ; q = 0 ;
   - lorsque M = Pd, alors :
      X=Cl; m=n=1 ; p = 2 ; q = 0 ; ou bien
      X = π-allyl ; m = n = p = 2 ; q = 0 ;
   - lorsque M = Ni, alors X = Cl, Br ou I ; m = n = 1 ; p = 2 ; q = 0.

Comme diamine chirale on peut, par exemple, citer la (R,R) et la (S,S)-1,2-diphényléthylène diamine.

Un aspect particulier de l'invention a pour objet les catalyseurs métalliques de formule (VII) :

[Mᵣ Lₛ Zₜ Wᵤ]Yᵥ (VII)

dans laquelle :
- M représente un métal choisi parmi le rhodium, le ruthénium, l'iridium, ou le palladium ou le cuivre ;
- L représente un composé (I) chiral ;
- Z, W, r, s, t, u et v sont définis comme suit :
   - lorsque M = Rh, alors Z = 1,5-cyclooctadiène ou norbornadiène ; Y = BF₄, ClO₄, PF₆, OTf ou BPh₄; r = s = t = v =1 ; u = 0 ;
   - lorsque M = Ru, alors :
      Z = Cl, Br ou I ; W = benzène ou *p*-cymène ; Y = Cl, Br ou I; r = s = t = u = v = 1 ;
      ou bien Y = BF₄, ClO₄, PF₆ ou BPh₄ ; r = s = 1 ; t = u = 0 ; v=2;
      ou bien Z = Cl ; Y = NH₂(C₂H₅)₂ ; r = s = 2 ; t =5 ; u = 0 ; v=1 ;
   - lorsque M = Ir, alors Z = 1,5-cyclooctadiène ou norbornadiène ; Y = BF₄, ClO₄, PF₆ ou BPh₄ ; r = s = v = 1; t = 1,u = 0 ;
   - lorsque M = Pd, alors Y = BF₄, ClO₄, PF₆ ou BPh₄ ; r = s = v = 1 ; t = u = 0 ;
   - lorsque M = Cu, alors Y = PF₆ ou ClO₄ ; r = *s* = v = 1 ; t = u = 0.

Les catalyseurs au rhodium ou au ruthénium et en particulier ceux choisis parmi ceux ci-dessous sont actuellement préférés :
- les composés de forme (VI) :
   où M = Ru et X = Br; m = n = 1; p = 2; q = 0;
   ou X = Cl; S = N(CH₂CH₃)₃; m = n = 1 ; p = 4; q = 1;
   ou X = Cl ; S = pyridine ; m = n = 1 ; p = q = 2;
- les composés de formule (VII) :
où M = Rh et Z = 1,5-cyclooctadiène ou norbornadiène ; Y = BF₄, ClO₄, PF₆, OTf ou BPh₄; r = s = t = v = 1 ; u = 0.

Les catalyseurs comprenant un ligand de formule (I) selon l'invention et un métal choisi parmi le rhodium, le ruthénium, le palladium, l'iridium, le nickel ou le cuivre, peuvent être préparés selon des procédés décrits dans la littérature bien connus de l'homme de l'art. On pourra notamment se référer à la demande de brevet publiée sous le numéro EP 850 945.

Les catalyseurs selon l'invention sont généralement préparés à partir d'un complexe métallique de départ, dont la nature varie suivant le métal sélectionné.

Dans le cas des catalyseurs au rhodium, le complexe de départ est, par exemple, l'un des composés suivants : Rh(cod)₂OTf; [Rh(cod)Cl]₂ où cod désigne le 1,5-cyclooctadiène ; Rh(acac)(CO)₂ où acac désigne l'acétylacétonate ; ou Rh(acac)(C₂H₄)₂.

Pour la préparation des catalyseurs au ruthénium, les complexes comme RuCl₃, Ru(cod)(methylallyl)₂, [RuCl₂(benzène)]₂, [RuCl₂(nbd)]ₓ avec nbd représentant le norbornadiène et x un entier, pourront être utilisés. On peut également citer le Ru(acac)₃ et le [RuCl₂(cod)]ₓ avec x représentant un nombre entier.

D'une manière générale, les catalyseurs métalliques selon l'invention sont préparés par mélange du complexe métallique de départ, d'un ligand de formule (I) et d'un solvant organique anhydre et dégazé et éventuellement, maintien du mélange réactionnel à une température comprise entre 15 et 150°C, de préférence entre 30 et 120°C, pendant, par exemple, 10 minutes à 5 heures.

A titre de solvant, on pourra utiliser les hydrocarbures aromatiques (tels que le benzène, le toluène ou le xylène), les amides (tels que le formamide, le diméthylformamide, le diméthylacétamide, la *N*-méthyl-2-pyrrolidinone ou l'hexaméthylphosphorylamide), les alcools (tels que l'éthanol, le méthanol, le *n*-propanol et l'isopropanol) et leurs mélanges, les cétones (telles que l'acétone, la méthyléthylcétone, la méthylisobutylcétone et la cyclohexanone), les éthers (comme par exemple le tetrahydrofurane), les alcanes linéaires ou ramifiés ou cycliques (tels que le pentane, l'hexane, le méthylcyclohexane).

Le catalyseur est ensuite isolé selon des techniques classiques (filtration ou cristallisation) et utilisé dans des réactions asymétriques. Néanmoins, le catalyseur peut également être préparé *in situ.* La réaction, devant être catalysée par le catalyseur ainsi préparé, peut alors être mise en oeuvre sans isolement intermédiaire du catalyseur.

La présente invention a également pour objet l'utilisation des catalyseurs métalliques selon la présente invention pour catalyser des réactions asymétriques, notamment d'hydrogénation et de formation de liaison C-C. Les procédés d'hydrogénation ou de formation de liaison C-C asymétrique mettant en oeuvre de tels catalyseurs font partie intégrante de l'invention. Ces procédés sont réalisés dans des conditions bien connues de l'homme de l'art.

Par exemple, dans le cas d'une réaction d'hydrogénation asymétrique, le substrat insaturé, en solution dans un solvant comprenant le catalyseur, est placé sous pression d'hydrogène. Les conditions opératoires sont analogues à celles couramment mises en oeuvre avec les catalyseurs métalliques de l'art antérieur. Par exemple, une pression d'hydrogène comprise entre 1 et 150 bar et une température de 0°C à 150°C seront utilisées. Le rapport molaire du substrat au catalyseur varie généralement de 1/100 à 1/100 000, de préférence de 1/100 à 1/5000.

Les complexes du rhodium préparés à partir des ligands de l'invention sont plus spécialement appropriés à la catalyse asymétrique des réactions d'isomérisation d'oléfines, d'hydrogénation de liaisons C=C et pour l'addition-1,4 d'acides boroniques.

Les complexes du ruthénium préparés à partir des ligands de l'invention sont plus spécialement appropriés à la catalyse asymétrique des réactions d'hydrogénation de liaisons carbonyle, de liaisons C=C et de liaisons C=N.

Dans les exemples qui suivent, on désigne par « préparation » les exemples décrivant la synthèse de composés intermédiaires et, par « exemple » ceux décrivant la synthèse de composés de formule (I), (VI) ou (VII) selon l'invention. Ces exemples ont pour but d'illustrer l'invention et ne sauraient, en aucun cas, en limiter la portée. Les points de fusion sont mesurés au banc Koffler et les valeurs spectrales de Résonance Magnétique Nucléaire sont caractérisées par le déplacement chimique δ calculé par rapport au TMS, par le nombre de protons associés au signal et par la forme du signal (s pour singulet, d pour doublet, t pour triplet, m pour multiplet, dd pour doublet de doublet, ddd pour doublet dédoublé dédoublé, q pour quadruplet, qd pour quadruplet dédoublé, J pour constante de couplage). La fréquence de travail et le solvant utilisé sont indiqués pour chaque composé. Les résultats de spectrométrie de masse sont obtenus avec un appareil Hewlett Packard 7989 A.

Les abréviations suivantes sont utilisées : TA = température ambiante, DMSO = diméthylsulfoxyde, Ph = phényle, THF = tétrahydrofuranne, Me = méthyle, Et = Ethyle, acac = acétylacétonate, Tf = triflate ; S-DPED = (S,S)-diphényléthylènediamine.

La nomenclature utilisée pour identifier les composés est celle préconisée par les Chemical Abstracts.

### PREPARATION 1

### 6-Bromo-2,3-dihydro-1,4-benzodioxine, composé V

35 g de 1,4-benzodioxane et 200 ml de diméthylformamide anhydre sont placés sous argon à 0°C. 54,9 g de *N*-bromosuccinimide sont alors ajoutés par portions. Après un retour progressif à température ambiante, le mélange réactionnel est agité pendant 24 heures. Les solvants sont évaporés sous pression réduite et le solide blanc obtenu est lavé au dichlorométhane. Le filtrat est traité avec 50 ml d'une solution aqueuse saturée en sulfate de sodium, lavé avec 50 ml d'une solution aqueuse saturée en chlorure de sodium et séché sur sulfate de magnésium. Après évaporation des solvants sous pression réduite, une huile jaune est obtenue (rendement quantitatif).
Spectre de masse IE : M^{.+} = 214
RMN ¹H (200MHz) CDCl₃ : 4,25 (4H, s) ; 6,74 (1H, d) ; 6,93 (1H, dd) ; 7,02 (1H, d)

### PREPARATION 2

### 6-Bromo-2,3-dihydro-1,4-benzodioxine, composé V

Le *composé V peut* également être préparé selon le mode opératoire suivant :
5 g de 1,4-benzodioxane et 100 ml de tétrahydrofurane anhydre sont placés sous argon à l'abri de la lumière. 5,12 g de *1,3*-dibromo-5,5-diméthylhydantoïne sont alors ajoutés. Le mélange réactionnel est agité à température ambiante à l'abri de la lumière pendant 18 heures. Après évaporation de la moitié du tétrahydrofurane, 50 ml de pentane sont ajoutés. Après filtration, l'opération est recommencée trois fois puis les solvants sont évaporés sous pression réduite. Le résidu huileux obtenu est purifié par chromatographie sur une colonne de gel de silice en éluant avec un mélange cyclohexane/acétate d'éthyle, 8/2, v/v (rendement = 90 %).

### PREPARATION 3

### (2,3-dihydro-1,4-benzodioxin-6-yl)diphénylphosphine, composé IVA.1

11 g de *composé V et* 30 ml de tétrahydrofurane anhydre sont placés sous argon et refroidis à - 78°C. 25,6 ml de *n*-butyllithium 2,2 M dans le dioxane sont ajoutés goutte à goutte puis le mélange réactionnel est agité à - 78° C pendant 1 heure. 10,4 ml de chlorodiphénylphosphine sont alors ajoutés goutte à goutte, en maintenant la température à - 60°C. La température du mélange réactionnel remonte alors lentement à 0°C et 20 ml d'une solution saturée en chlorure d'ammonium sont additionnés à 0°C. La phase organique est alors lavée avec 2 fois 20 ml d'une solution saturée en chlorure de sodium, séchée sur sulfate de magnésium, filtrée et les solvants sont évaporés sous pression réduite pour donner une huile orange qui cristallise. Le solide est ensuite lavé à l'hexane chaud puis filtré (rendement = 90%).
RMN ¹H (200MHz) CDCl₃ : 4,26 (4H, m) ; 6,80-6,84 (2H, m) ; 6,85 (1H ; d : J=4 Hz) ; 7,32 (10H, m)
RMN ³¹P (162 MHz) CDCl₃ :-4,66 ppm

### PREPARATION 4

### (2,3-dihydro-1,4-benzodioxin-6-yl)diphénylphosphine oxyde, composé IIIA.1

A une suspension de 16,3 g de *composé IVA.1* dans 60 ml de méthanol, 8 ml d'une solution aqueuse d'eau oxygénée à 30 % sont additionnés goutte à goutte, tout en maintenant la température du mélange réactionnel inférieure à 40°C. Après une heure d'agitation, 14 ml d'une solution aqueuse de sulfite de sodium à 30% sont ajoutés. L'agitation est maintenue pendant 1 heure, puis 9 ml d'une solution aqueuse d'acide chlorhydrique 1N sont ajoutés. La solution est concentrée à 40° C et le résidu aqueux est extrait avec 50 ml de dichlorométhane. La phase organique est séchée sur sulfate de magnésium et les solvants sont évaporés sous pression réduite pour donner une huile jaune qui cristallise. Le solide obtenu est lavé à l'hexane chaud puis filtré (rendement quantitatif).
Spectre de masse IE : M⁺ = 335
RMN ¹H (200MHz) CDCl₃ : 4,26 (4H,m) ; 6,95 (1H , dd : J = 11,8 Hz, J= 3,1 Hz) ; 7,09-7,18 (2H, m) ; 7,42-7,54 (6H, m) ; 7,61-7,72 (4H, m)
RMN³¹P (162 MHz) CDCl₃ : 30,1 ppm

### PREPARATION 4 bis

**(2,3-dihydro-1,4-benzodioxin-6-yl)diphénylphosphine oxyde, composé IIIA.1** A une suspension de 12,4 g de magnésium dans 31 ml de THF anhydre, sont ajoutés, sous azote, en 1 heure environ, 100,0g de 6-bromo-2,3-dihydro-1,4-benzodioxine dilués dans 200ml de THF anhydre en maintenant la température en dessous de 60°C. Après maintien du mélange réactionnel à 60°C pendant 2 heures sont additionnés en 3 heures 107,7g de chlorodiphénylphosphine en ne dépassant pas 10°C dans le milieu réactionnel. Après 18 heures de maintien à 20°C sont ajoutés 35 ml de méthanol. Le milieu réactionnel est agité pendant une heure puis refroidi à 0°C. 30 ml de peroxyde d'hydrogène à 35% sont alors ajoutés en ne dépassant pas 5°C dans le mélange réactionnel. Après maintien pendant 2 heures à 20°C et évaporation des solvants sous pression réduite, le solide obtenu est dissous à chaud dans 900 ml d'acétate d'isopropyle, puis successivement lavé par 3 fois 200 ml d'HCl 1N, 150 ml d'une solution aqueuse de carbonate de potassium 1N, 150 ml d'eau puis séché sur sulfate de magnésium. Après évaporation de 500 ml de solvant sous pression réduite, le mélange réactionnel est refroidi à 0°C, filtré, et le solide est rincé par 2 fois 30 ml d'acétate d'isopropyle. Après séchage pendant 72 heures à 20°C sous pression réduite, on obtient 113 g d'un solide blanc crème (rendement 72%).

### PREPARATION 5

### (S)-[5,5'-bi-(2,3-dihydro-1,4-benzodioxin)-6,6'-diyl]bis(diphénylphosphine oxyde), composé IIA.1

30 g de *composé IIIA.1* et 600 ml de tétrahydrofurane anhydre sont dégazés, placés sous argon puis refroidis à - 100°C, à l'aide d'un cryostat. 65 ml d'une solution de *tert*-butyllithium à 1,5 M dans le pentane sont ajoutés, goutte à goutte, à - 100°C. Le mélange réactionnel est porté à - 70°C sur 30 minutes, puis agités à cette température pendant 3 heures 30 minutes. 19,8 g de trichlorure de fer anhydre sont alors ajoutés, en une portion, sous courant d'argon. La température est ensuite lentement amenée à température ambiante et le mélange réactionnel est agité pendant 12 heures. Le mélange réactionnel est concentré à 60°C et 50 ml d'une solution aqueuse d'hydroxyde de sodium 1N et 500 ml de dichlorométhane sont additionnés. Le précipité obtenu est filtré sur célite, puis rincé avec 100 ml de dichlorométhane. La phase organique est lavée avec 50 ml d'eau, 50 ml d'une solution aqueuse saturée en chlorure de sodium et séchée sur sulfate de magnésium. Après évaporation des solvants sous pression réduite, le solide obtenu est dissous dans 150 ml de chloroforme et 12 g d'acide(-)-L-dibenzoyltartrique en solution dans 180 ml d'acétate d'éthyle sont alors ajoutés. Au bout de quelques minutes, un précipité apparaît. Ce précipité est essoré, puis est mis en suspension dans 200 ml de dichlorométhane et 100 ml d'une solution aqueuse d'hydroxyde de potassium 1N sont additionnés. Le mélange réactionnel est agité, à température ambiante, pendant 30 minutes, puis la phase organique est séparée, lavée avec 50 ml d'eau, 50 ml d'une solution aqueuse saturée en chlorure de sodium, puis séchée sur sulfate de magnésium. Les solvants sont alors évaporés sous pression réduite (rendement = 50 %).
RMN ¹H (200MHz) CDCl₃ : 3,42 (2H, m) ; 3,69 (2H, m) ; 3,92 (2H, m) ; 4,06 (2H, m) ; 6,65 (2H, dd) ; 6,77 (dd) ; 7,26-7,56 (16H, m) ; 7,68 (4H,m)
RMN ³¹P (162 MHz) CDCl₃ : 30,97 ppm
Spectre de masse IC : MH^{·+} = 671
Point de fusion > 260°C
[α]_{D}²⁰ (CHCl₃ , C=1) = - 140°.

Une structure RX du complexe du composé IIA.1 avec l'acide L-dibenzoyltartrique a démontré la configuration absolue (S).

Selon un mode opératoire analogue, **on prépare *le composé IIA.2* : (R)-[ 5,5'-bi (2,3-dihydro-1,4-benzodioxin)-6,6'-diyl]bis(diphénylphosphine oxyde)**
[α]_{D}²⁰ (CHCl₃ , C=1) = + 143 °.

### PREPARATION 6

### (2,2-Difluoro-1,3-benzodioxol-5-yl)diphénylphosphine oxyde, composé IIIA. 2

A une suspension de 10,2 g de magnésium dans 25,2 ml de THF anhydre, sont ajoutés, sous azote, en 2 heures, 90,1 g de 5-bromo-2,2-difluoro-1,3-benzodioxole dilués dans 168 ml de THF anhydre en maintenant la température à 60°C. Après maintien du mélange réactionnel à température ambiante pendant 3 heures, sont additionnés en 2 heures, 90 g de chlorodiphénylphosphine oxyde en ne dépassant pas 20°C dans le mélange réactionnel. Après 19 heures de maintien à 20°C, le mélange réactionnel est hydrolysé avec 27 ml d'eau, 135 ml d'HCl 1N puis extrait par 270 ml d'acétate d'éthyle. Après décantation et séparation des phases, la phase organique est successivement lavée avec 135 ml d'HCl 1N, 135 ml d'une solution aqueuse saturée de bicarbonate de potassium, 135 ml d'eau puis séchée sur sulfate de sodium. Les solvants sont évaporés sous pression réduite pour donner 128 g d'une huile visqueuse marron. Cette huile est purifiée par filtration sur silice en éluant avec un mélange acétate d'éthyle/heptane variant de 50/50 à 100/0, v/v (huile brune, 90 g, rendement = 66%).
Spectre de masse IC : MH^{.+} = 359
RMN ¹H (300MHz) CDCl₃ : 7,70-7,41 (11H, m) ; 7,37 (1H, dd); 7,16 (1H, dd)

### PREPARATION 7

### (2,2-difluoro-4-iodo-1,3-benzodioxol-5-yl)diphénylphosphine oxyde, composé IIIC.1

A une solution de 35,5 ml de diisopropylamine diluée dans 150 ml de THF anhydre, sont ajoutés sous azote en 40 minutes à 0°C, 96,6 ml de butyllithium 2,5 M en solution dans l'hexane. Après 15 minute d'agitation à 0°C, la solution est additionnée lentement sous azote en 1 heure, sur une solution de 82,5 g du *composé IIIA.2* à -78°C dilués dans 600 ml de THF anhydre puis l'agitation est maintenue à -78°C pendant 50 minutes. A la solution précédente à -78°C , est additionnée en une heure, une solution de 60,9 g d'iode dilués dans 250 ml de THF anhydre. Le mélange réactionnel est ensuite lentement amené à température ambiante puis agité pendant 20 heures. Le mélange réactionnel est ensuite refroidi à 0°C, filtré, et le solide rincé par 3x20 ml de THF. Après séchage pendant 5 heures à 40° C sous pression réduite, on obtient 97,6 g d'un solide blanc crème (rendement = 87,5%).
Spectre de masse IE : M^{.+} = 484
RMN ¹H (250MHz) CDCl₃ : 7,73-7,48 (10H, m) ; 7,04-6,96 (2H, m)

### PREPARATION 8

### (R,S)-[4,4'-bi(2,2-difluoro-1,3-benzodioxole)-5,5'-diyl]bis(diphénylphosphine oxyde) composé IIA.3 racémique

30 g de *composé IIIC.1,* 11,8 g de cuivre en poudre et 150 ml de DMF sont chauffés à 130°C pendant 4 heures. Le mélange réactionnel est ensuite amené à température ambiante, filtré, puis concentré. L'huile marron obtenue est alors diluée dans 300 ml de dichlorométhane, puis lavée successivement par 100 ml d'une solution aqueuse saturée de chlorure d'ammonium, 100 ml d'eau et séchée sur sulfate de magnésium. Le solide jaune obtenu est ensuite recristallisé dans 250 ml de méthanol à 0°C pour donner après séchage sous pression réduite 15,2 g d'un solide blanc (rendement = 68,7%).
Spectre de masse IC : M⁺ = 715
RMN ¹H (300MHz) CDCl₃ : 7,66-7,25 (20H, m) ; 7,03-7,00 (4H, m)

Le *composé IIA.3* est ensuite dédoublé par chromatographie sur une colonne à phase chirale commercialisée sous le nom Chirose® C3 pour fournir les énantiomères (S) et (R) optiquement purs.

### PREPARATION 9

### Acide (2,3-dihydro-1,4-benzodioxin-6-yl)phosphonique, diphényl ester (Composé IIIB.1)

On place dans un ballon à trois cols, sous argon, 602 mg (25,6 mM) de magnésium activé et 1 ml de tétrahydrofurane anhydre (THF). On ajoute deux gouttes de 1,3-dibromopropane, puis, tout en maintenant la température à 0°C, 5 g (23,3 mM) du composé V en solution dans 10 ml de THF. Le mélange réactionnel est agité pendant 2 heures à température ambiante puis 1 heure à reflux du solvant. Le magnésien formé est ensuite ajouté lentement à une solution de 4,84 ml (23,25 mM) de chlorure diphénylphosphinique dans 5 ml de THF préalablement refroidie à -5°C. La solution est agitée pendant une nuit à température ambiante, puis concentrée sous pression réduite. Le résidu est repris dans 20 ml d'acétate d'éthyle et agité avec 10 ml d'une solution normale d'acide chlorhydrique pendant 30 minutes. La phase aqueuse est extraite à l'acétate d'éthyle, les phases organiques rassemblées sont lavées à l'eau, séchées sur sulfate de magnésium et concentrées sous pression réduite. L'huile obtenue est purifiée par chromatographie sur gel de silice en éluant à l'aide d'un mélange cyclohexane-acétate d'éthyle (7/3 ;v/v). On obtient ainsi 5 g du produit attendu sous forme d'un solide blanc rosé (Rendement = 59 %).
RMN ¹H (200 MHz, CDCl₃): *δ =* 4,25-4,32 (m, 4H), 6,95 (dd, *J =* 5,1, 8,1 Hz, 1H), 7,10-7,35 (m, 10H), 7,39-7,43 (m, 1H), 7,47 (ddd, 1H) ;
RMN ¹³C (50 MHz, CDCl₃) : δ= 64,1, 64,5, 117,7 (d, *J =* 18,5 Hz), 120,5 (d, J = 4,5 Hz), 121,6 (d, *J* = 12,7 Hz), 124,9, 125,9 (d, *J* = 10,6 Hz), 129,6, 143,5 (d, J = 22,2 Hz), 147,9, 150,5 (d, J = 7,4 Hz) ;
RMN ³¹P (162 MHz, CDCl₃) : δ = 13,11.
Spectre de masse (IE) : M^{.+} = 368.

### PREPARATION 10

### Acide (2,3-dihydro-1,4-benzodioxin-6-yl)phosphonique, diéthyl ester (Composé IIIB.2)

On place 20 g (92,8 mM) du composé obtenu selon la préparation 1 et 1,2 g (9,28 mM) de chlorure de nickel dans un ballon muni d'un appareillage de distillation. Le mélange est agité et porté à 160°C et on ajoute goutte à goutte 18,8 ml (111,4 mM) de triéthylphosphite. Le mélange réactionnel est agité à 160°C pendant une heure après la fin de l'addition, tandis que le bromoéthane formé par la réaction est collecté par distillation. Le milieu réactionnel est ensuite refroidi et on ajoute 50 ml d'éther éthylique et 50 ml d'acétate d'éthyle. La suspension obtenue est filtrée et le filtrat est concentré sous pression réduite. Le résidu est ensuite purifié par chromatographie sur gel de silice en éluant à l'aide d'acétate d'éthyle, pour donner 25 g du produit attendu sous forme d'une huile incolore (Rendement quantitatif).
RMN ¹H (400 MHz, CDCl₃): δ= 1,24 (t, *J =* 7,0 Hz, 6H), 4,01 (qd, *J =* 7,0, 9,9 Hz, 4H), 4,20-4,23 (m, 4H), 6,86 (dd, *J* = 8,1, 4,6 Hz, 1H), 7,19-7,22 (m, 2H) ;
RMN ¹³C (50 MHz, CDCl₃ ) : δ = 16,1, 61,8, 64,0, 64,4, 117,5 (d, *J* = 17,5 Hz), 120,9 (d, *J* = 12,0 Hz), 125,3 (d, *J =* 10,0 Hz), 125,5, 143,4 (d, J = 20,8 Hz), 147,2 ;
RMN ³¹P (162 MHz, CDCl₃) : δ= 20,20.
Spectre de masse (IE) : M^{.+} = 272.

### PREPARATION 11

### Acide [5,5'-bi(2,3-dihydro-1,4-benzodioxin)-6,6'-diyl]diphosphonique, tétraphényl ester (Composé IIB.1)

On prépare une solution de 0,675 ml (3,97 mM) de tétraméthylpiperidine dans 5 ml de THF que l'on refroidit à -78 °C et on ajoute 1,32 ml (3,24 mM) d'une solution 2,4 M de n-butyllithium dans l'hexane. La solution est agitée pendant 30 min à -15 °C puis ajoutée à une solution de 1 g (2,27 mM) du composé IIIB.1 dans 5 ml de THF refroidie à -78 °C. Le mélange est agité pendant 1 heure à - 78 °C, puis on ajoute 570 mg (3,5 mM) de chlorure ferrique anhydre. Le mélange est agité pendant une nuit à température ambiante, puis concentré sous pression réduite. Le résidu est repris dans 30 ml de dichlorométhane et agité pendant 30 min en présence de 15 ml d'une solution de soude N. Le mélange est filtré et la phase organique séparée est lavée avec successivement 15 ml d'eau, 15 ml d'une solution d'acide chlorhydrique N, 10 ml d'eau et 10 ml d'une solution saturée de chlorure de sodium. Cette phase organique est ensuite séchée sur sulfate de magnésium et concentrée sous pression réduite. Le résidu est purifié par chromatographie sur gel de silice en éluant avec un mélange cyclohexane/acétate d'éthyle (1/1; v/v) pour donner 200 mg du produit attendu sous forme d'un solide jaune pâle (Rendement = 20%).
RMN ¹H (200 MHz, CDCl₃): δ = 3,80-3,90 (m, 2H), 3,95-4,10 (m, 4H), 4,14-4,30 (m, 2H), 6,88 (dd, J = 8,2, 17,7, 8H), 7,00-7,22 (m, 14H), 7,72 (dd, *J* = 14,5, 8,4 Hz, 2H) ; RMN ¹³C (50 MHz, CDCl₃) : δ= 63,8, 64,2, 116,8 (d, *J* = 18,5 Hz), 120,8, 124,5, 126,9 (d, *J* = 9,8 Hz), 129,2, 142,3 (d, J = 22,1 Hz), 147,6 , 150,5 (d, J = 8,1 Hz) ;
RMN ³¹P (162 MHz, CDCl₃) : δ = 11,68.
Spectre de masse (IC) : (M+H)⁺ = 735.

### PREPARATION 12

### Acide [5,5'-bi(2,3-dihydro-1,4-benzodioxin)-6,6'-diyl]diphosphonique, tétraéthyl ester (Composé IIB.2)

On prépare une solution de 6,72 ml (44,4 mM) de TMEDA (tétraméthyléthylènediamine) et 5 g (18,5 mM) du composé IIIB.2 dans 50 ml de THF et on ajoute, à - 60°C, 20,2 ml (22,2 mM) d'une solution 1,1 M de *sec*-butyllithium dans l'hexane. La solution est agitée pendant 2h à -60°C puis 3,91g de chlorure ferrique anhydre (24 mM) sont ajoutés en une portion à -60°C. Le mélange est agité pendant une nuit à température ambiante. Le mélange est concentré sous pression réduite, repris dans 100 ml de dichlorométhane et 30 ml d'une solution de soude 1N et la suspension est agitée pendant 30 min. Après filtration, la phase organique est lavée à l'eau, puis avec successivement 30 ml d'une solution d'acide chlorhydrique N, 30 ml d'eau, 30 ml d'une solution saturée de chlorure de sodium, séchée sur sulfate de magnésium, filtrée et concentrée sous pression réduite. Le résidu solide est cristallisé (éther éthylique/hexane, 1:1) et on obtient 1,6 g du composé attendu sous forme d'un solide blanc (Rendement = 32%).
RMN ¹H (400 MHz, CDCl₃): δ = 1,13 (q, *J* = 7,0 Hz, 12H), 3,69-3,80 (m, 2H), 3,85-3,92 (m, 6H), 4,14-4,17 (m, 4H), 4,22-4,24 (m, 4H), 6,90 (dd, *J =* 4,0, 8,3. Hz, 2H), 7,41 (dd, *J* = 13,8, 8,5 Hz, 2H) ;
RMN ¹³C (50 MHz, CDCl₃):δ = 16,1, 61,2 (d, J = 8,0 Hz), 63,9, 64,2, 116,2 (d, *J* =17,3 Hz), 125,2, 125,9 (d, *J* = 9,1 Hz), 128,8 (d, J = 12,1 Hz), 141,8 (d, J = 20,8 Hz), 146,5 ; RMN ³¹P (162 MHz, CDCl₃) : δ = 19,13.
Spectre de masse (IE) : M^{.+} = 542.

### PREPARATION 13

### Tétrachlorure [5,5'-bi(2,3-dihydro-1,4-benzodioxin)-6,6'-diyl] diphosphonique, (Composé IIC)

2 g (3,69 mM) du composé IIB.2, 16 ml de chlorure de thionyle et 0,4 ml de diméthylformamide sont introduits dans un ballon surmonté d'un réfrigérant sous atmosphère d'argon et portés au reflux (80-90°C) pendant 4,5 h. La solution devient jaune vif. Le mélange est concentré sous pression réduite et séché pour donner un solide orange foncé (aiguilles) qui peut être conservé au réfrigérateur sous atmosphère d'argon, avant d'être utilisé directement dans l'étape suivante.
RMN ¹H (400 MHz, CDCl₃): δ = 4,17-4,36 (m, 8H), 7,06 (dd, *J* = 8,7, 5,8 Hz, 2H), 7,54 (dd, *J* = 20,0, 8,7 Hz, 2H) ;
RMN ³¹P (162 MHz, CDCl₃): δ = 34,74.

### PREPARATION 14

### [5,5'-bi(2,3-dihydro-1,4-benzodioxin)-6,6'-diyl]bis[di(4-méthylphényl) phosphine oxyde], composé IIA.4

A une solution de 6,3 g (36,9 mM) de 4-bromotoluène dans 50 ml de THF sont ajoutés, à -78°C, 19,9 ml (36,9 mM) d'une solution de n-butyllithium 1,85 M dans l'hexane. Une suspension blanche apparaît. La solution est agitée pendant 1h à -78°C, puis ajoutée à une solution de 1,86 g (3,69 mM) du composé IIC dans 10 ml de THF. La solution devient marron foncé. Le mélange est alors amené à température ambiante puis agité pendant 1 h à 50°C. 20 ml d'une solution saturée de chlorure d'ammonium sont ajoutés et la phase organique est lavée à l'eau, puis avec une solution saturée de chlorure de sodium, séchée sur sulfate de magnésium et concentrée sous pression réduite. Le résidu est purifié par chromatographie sur gel de silice en éluant avec un mélange acétate d'éthyle/méthanol (9/1 ;v/v). On obtient ainsi 1,32 g du composé attendu sous forme d'un solide beige (Rendement = 50% sur deux étapes).
RMN ¹H (400 MHz, CDCl₃):δ = 2,30 (s, 6H), 2,38 (s, 6H), 3,57 (ddd, J = 2,3, 7,2, 11,4 Hz, 2H), 3,75 (ddd, J = 2,4, 4,3, 11,6 Hz, 2H), 3,97 (ddd, J = 2,3, 4,2, 11,2 Hz, 2H), 4,09 (ddd, J = 2,6, 7,2, 11,1 Hz, 2H), 6,65 (dd, J = 8,5, 13,2 Hz, 2H), 6,74 (dd, J = 3,0, 8,4 Hz, 2H), 7,04 (dd, J = 2,4, 8,0 Hz, 4H), 7,20 (dd, J = 2,1, 8,0 Hz, 4H), 7,32 (dd, J = 8,0, 11,8 Hz, 4H), 7,53 (dd, J = 8,0, 11,4 Hz, 4H).
RMN ¹³C (50 MHz, CDCl₃): δ = 21,4, 63,4, 64,0, 115,8, 126,5 (d, J = 13,3 Hz), 128,4 (d, J = 12,3 Hz), 132,1 (d, J = 10,4 Hz), 132,4, 132,9, 135,8, 140,9, 142,5, 145,7.
RMN ³¹P (162 MHz, CDCl₃): δ = 30,95.
Spectre de masse (IC) : (M+H)⁺ = 727.

### PREPARATION 15

### [5,5'-bi(2,3-dihydro-1,4-benzodioxin)-6,6'-diyl]bis[bis(3,5-diméthylphényl) phosphine oxyde], composé IIA.5

Ce composé est obtenu en opérant de façon analogue à la préparation 13, au départ de 5-bromo-*m*-xylène.
RMN ¹H (400 MHz, CDCl₃): δ= 2,12 (s, 12H), 2,31 (s, 12H), 3,63-3,66 (m, 2H), 3,75-3,79 (m, 2H), 4,00-4,04 (m, 2H), 4,08-4,13 (m, 2H), 6,72-6,75 (m, 4H), 6,96 (s, 2H), 7,11 (d, J = 12,7 Hz, 6H), 7,29 (d, J = 12,0 Hz, 4H) ;
RMN ¹³C (50 MHz, CDCl₃) : δ= 21,0, 21,3, 63,4, 63,9, 115,9 (d, J = 15,4 Hz), 126,6 (d, J = 12,9 Hz), 129,8, 132,5, 134,1, 136,1, 137,1, 141,0 (d, J = 14,9 Hz), 145,5 ;
RMN ³¹P (162 MHz, CDCl₃): δ = 31,78.
Spectre de masse (IC) : (M+H)⁺ = 783.

### EXEMPLE 1

### (S)-[5,5'-bi(2,3-dihydro-1,4-benzodioxin)-6,6'-diyl]bis(diphénylphosphine), composé I.1.

A 500 mg de *composé IIA.1* et 5 ml de xylène distillé dégazé, placés sous argon, 2,12 ml de tributylamine puis 780 µl de trichlorosilane sont ajoutés. Le mélange réactionnel est chauffé à 140°C pendant 12 heures. Une fois la température revenue à température ambiante, 5 ml d'une solution aqueuse d'hydroxyde de sodium 4N sont ajoutés. Le mélange réactionnel est ensuite agité, à température ambiante, pendant 30 minutes et 15 ml de dichlorométhane sont ajoutés. La phase organique est lavée avec 5 ml d'eau distillée, puis avec 5 ml d'une solution aqueuse saturée en chlorure de sodium, puis concentrée sous pression réduite. 10 ml de méthanol sont alors ajoutés et le précipité blanc obtenu est essoré sous argon, lavé avec 10 ml de méthanol puis séché sous pression réduite, pendant 4 heures (rendement = 91 %).
[α]_{D}²⁰ (benzène, C=0,1) = - 44°

De la même manière, on prépare les composés suivants :

### (R)-[5,5'-bi(2,3-dihydro-1,4-benzodioxin)-6,6'-diyl]bis(diphénylphosphine), composé I.2

RMN ¹H (200MHz) CDCl₃ : 3,35 (2H,m) ; 3,83 (4H,m) ; 4,13 (2H, m) ; 6,62 (2H, dd : J= 8 Hz , J=3 Hz) ; 6,85 (2H, d : J=8 Hz) ; 7,09 (4H, m) ; 7,23 (8H, m) ; 7,31 (8H, m)
RMN ³¹P (162 MHz) CDCl₃ : - 14,3 ppm
[α]_{D}²⁰ (benzène, C=0,1) = + 44°

### (R)-[4,4'-bi(2,2-difluoro-1,3-benzodioxole)-5,5'-diyl]bis(diphénylphosphine), composé I.3

[α]_{D}²⁰ (CH₃OH, C=0,5) = + 48°

### (S)-[4,4'-bi(2,2-difluoro-1,3-benzodioxole)-5,5'-diyl]bis(diphénylphosphine), composé I.4

[α]_{D}²⁰ (CH₃OH, C=0,5) = -49°

### [5,5'-bi(2,3-dihydro-1,4-benzodioxin)6,6'-diyl]bis[di(4-méthylphényl)phosphine), composé I.5.

RMN ¹H (200 MHz, CDCl₃) : δ = 2,29 (s, 6H), 2,33 (s, 6H), 3,32-3,44 (m, 2H), 3,74-3,94 (m, 4H), 4,03-4,18 (m, 2H), 6,62 (m, 2H), 6,82 (d, J = 8,4 Hz, 2H), 6,87-7,23 (m, 16H)

### [5,5'-bi(2,3-dihydro-1,4-benzodioxin)-6,6'-diyl]bis[bis(3,5-diméthylphényl)phosphine], composé I.6.

RMN ¹H (400 MHz, CDCl₃) δ= 2,12 (s, 12H), 2,25 (s, 12H), 3,47 (ddd, J = 2,3, 4,3, 12,0, 2H), 3,83 (ddd, J = 2,1, 6,9, 11,6, 2H), 3,95 (ddd, J = 2,2, 4,5, 11,4, 2H), 4,14 (ddd, J = 2,3, 7,2, 11,2, 2H), 6,63-6,67 (m, 6H), 6,80 (m, 4H), 6,90 (d, J = 8,1 Hz, 6H) ; RMN ³¹P (162 MHz, CDCl₃): δ = -14,52.

### EXEMPLE 2

### Composé VII.1 : complexe [Ru₂ Cl₅ L₂]⁻[(C₂H₅)₂NH₂]⁺ avec L = composé I.1

50 mg de bis(benzènedichlororuthénium), 128 mg de *composé I.1* et 21,6 mg de chlorhydrate de diéthylamine dans 10 ml de tétrahydrofurane sont portés à reflux pendant 16 heures. Les solvants sont alors évaporés sous pression réduite. Le solide obtenu est séché sous pression réduite.
RMN ³¹P (162 MHz) CDCl₃ : 51, 1(d) ; 54,4 (d) J= 38 Hz

### EXEMPLE 3

### Composé VI.1 : Complexe [LRuCl₂(pyridine)₂] avec L= composé I.2

42,2 mg de (norbomadiène)RuCl₂(pyridine)₂, 63,9 mg de *composé I.2* et 15 ml de CH₂Cl₂ anhydre dégazé sont alors ajoutés et le mélange réactionnel est agité pendant 12 heures sous argon à température ambiante. La solution est concentrée et séchée sous pression réduite pour donner 96 mg d'un solide jaune orange.
RMN ³¹P (162 MHz) CDCl₃ : 40,9 ppm

### EXEMPLE 4

### Composé VI.2: Complexe [LRuCl₂ S-DPED] avec L= composé I.2

34 mg de [LRuCl₂(pyridine)₂] obtenu à l'EXEMPLE 3, 7,4 mg de (S,S)-diphényléthylènediamine et 5 ml de CH₂Cl₂ anhydre dégazé sont agités pendant 2 heures sous argon à température ambiante. La solution est concentrée et séchée sous pression réduite pour donner 96 mg d'un solide jaune orange.
RMN ³¹P (162 MHz) CDCl₃ : 48,1 ppm

### EXEMPLE 5

### Composé VII.2 : Complexe [LRhcod]⁺ BF₄⁻ avec L = composé I.2

50 mg de [Rh(cod)₂]⁺ BF₄⁻ et 58,6 mg de *composé I.2* sont placés dans un schlenk. Le système est mis sous argon par 3 purges vide/argon successives. 10 ml de THF sont alors ajoutés et le mélange réactionnel est agité pendant 30 minutes. Après évaporation du solvant, le résidu obtenu est séché sous vide et on obtient 110 mg d'une poudre jaune.

### EXEMPLE 6

### Hydrogénation asymétrique

### Méthodes générales :

**a)** avec des catalyseurs chiraux du ruthénium préparés *in situ* de formule [LRuBr₂] avec L = composé (I)
   A 3,2 mg de (1,5-cyclooctadiène)bisméthylallyliuthénium et 1,1 équivalent de composé (I) dans 1 ml d'acétone sous argon, sont additionnés goutte à goutte 2,2 équivalents d'une solution d'acide bromhydrique à 0,16 N - 0,19 N dans le méthanol. Après 30 minutes d'agitation à température ambiante, les solvants sont évaporés sous pression réduite.
   Le substrat à hydrogéner (1 mmole) est alors dissous dans 2 ml de solvant d'hydrogénation (de type alcool ou halogéné tel que le dichlorométhane) et placé dans un autoclave en présence du catalyseur sous la pression désirée d'hydrogène et à la température désirée.
**b)** avec le trichlorure de ruthénium
   Le substrat à hydrogéner (1 mmole) dissous dans 2 ml de solvant d'hydrogénation est additionné à 2,1 mg de trichlorure de ruthénium et 1,1 équivalent de composé (I). L'hydrogénation est réalisée dans un autoclave à la pression et à la température désirées pendant le temps nécessaire.
**c)** avec le complexe décrit à l'EXEMPLE 2
   Le substrat à hydrogéner (1 mmole) dissous dans 2 ml de solvant d'hydrogénation est additionné à 3,6 mg de complexe. L'hydrogénation est réalisée dans un autoclave à la pression et à la température désirées pendant le temps nécessaire.
   Les catalyseurs selon l'invention pour hydrogénation stéréosélective sont utiles pour réaliser des réductions du type :

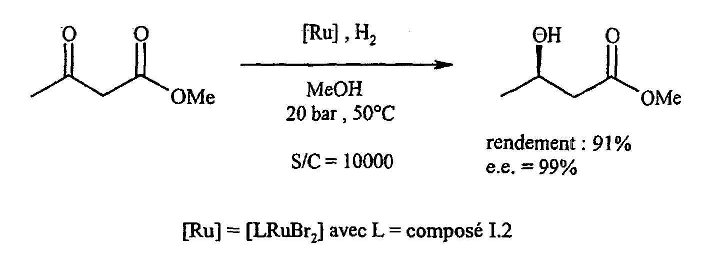

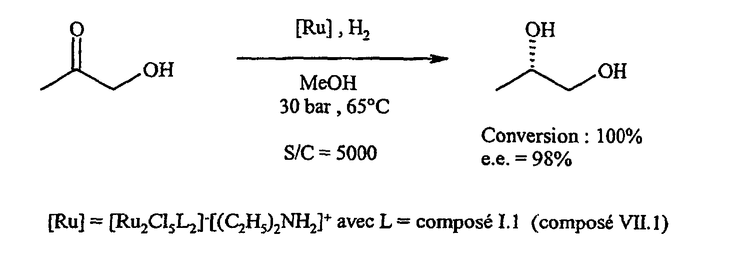

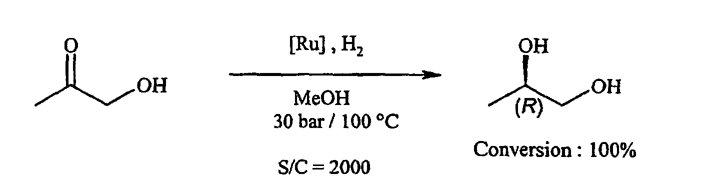

| **[Ru]** | **e.e. (%) (config)** |
|---|---|
| [LRuBr₂] *in situ* | 98,5 (*R*) |
| Composé VII.1* : [Ru₂Cl₅L₂]₋[(C₂H₅)₂NH₂]⁺ | 98,2 (*R*) |

| | |
|---|---|
| *avec L = composé I.2 | |

S/C représente le rapport pondéral substrat/catalyseur

Les excès énantiomériques (e.e.) obtenus par hydrogénation de différents substrats sont présentés dans le **TABLEAU 1** ci-après à titre d'exemples, les conditions utilisées n'étant pas optimisées (les indications portées dans la colonne [Ru] se référent à la méthode de préparation du catalyseur).

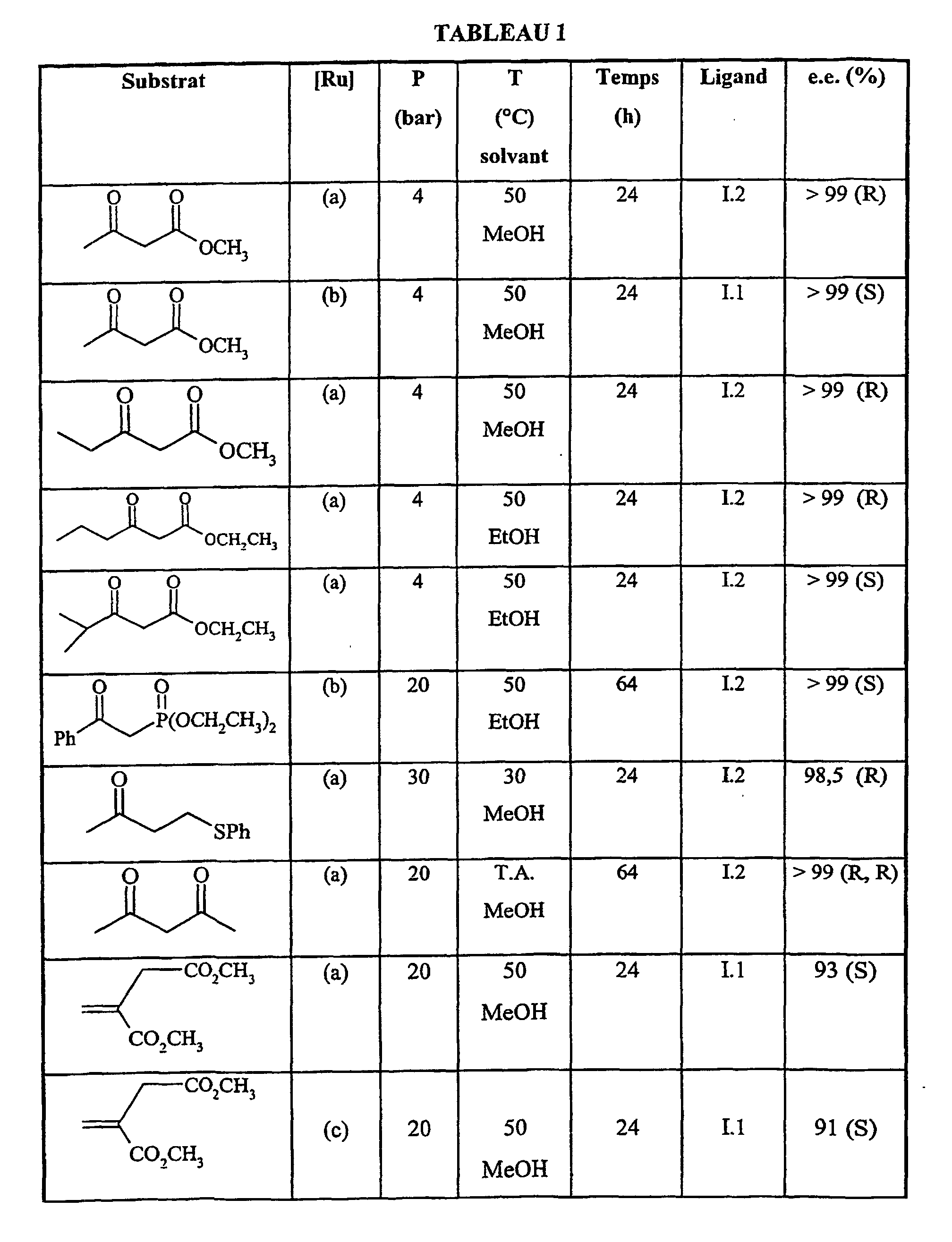

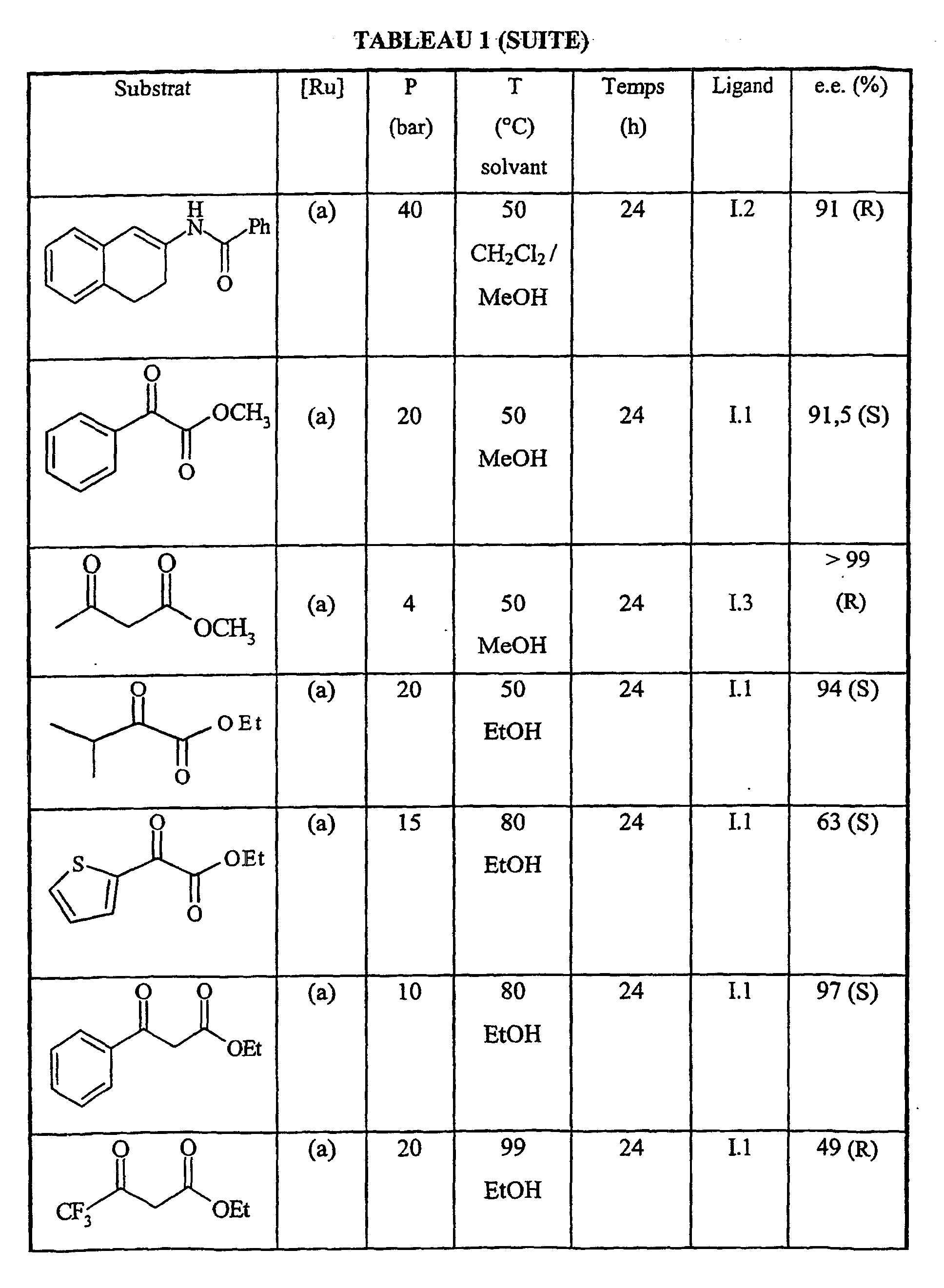

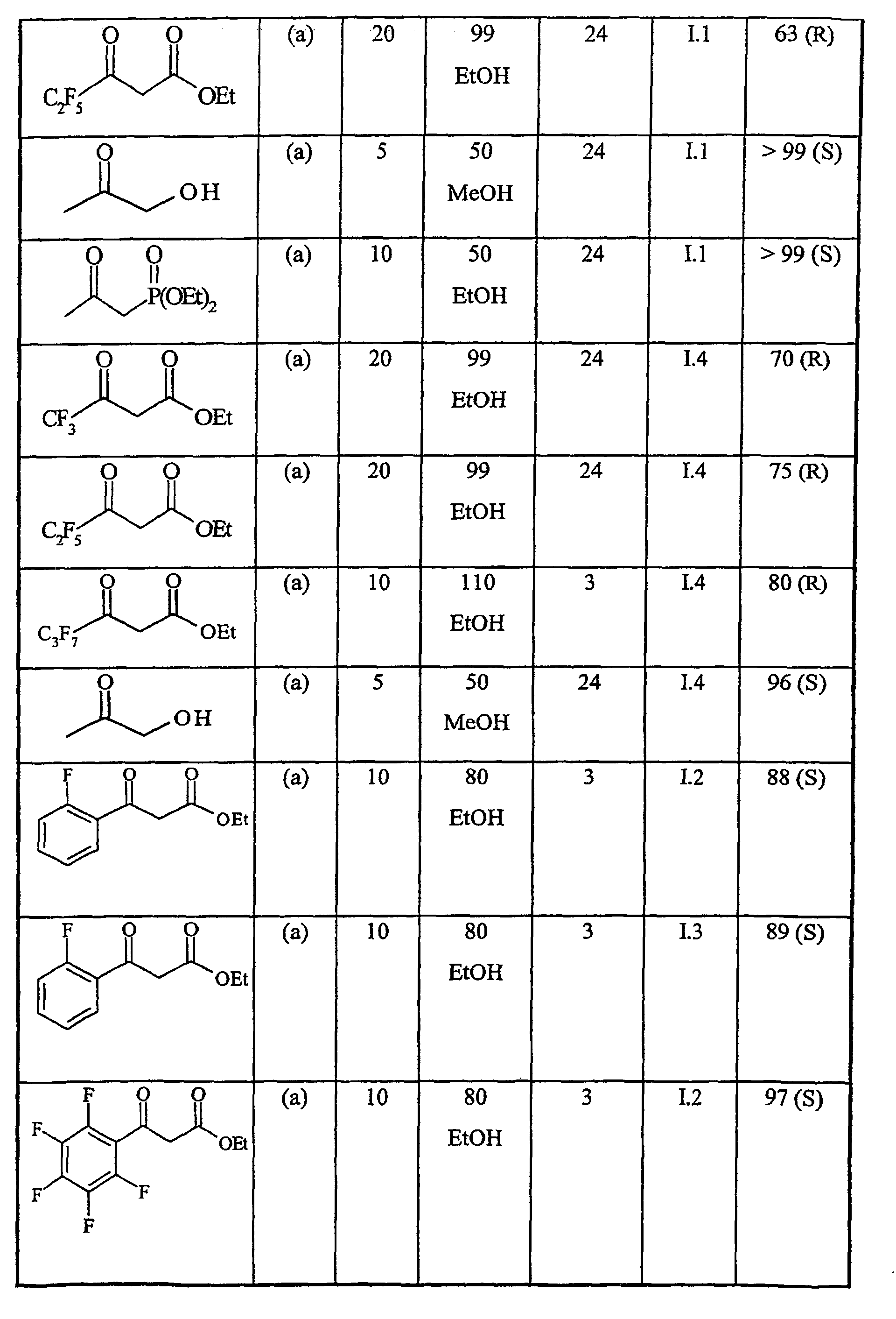

### EXEMPLE 7

### Addition 1,4-asymétrique

A 3,1 mg de Rh(acac)(C₂H₄)₂, 0,012 mmol de *composé I.1* et 2 mmol d'acide phénylboronique, sous argon, on additionne 1 ml de dioxane, 0,1 ml d'eau distillée et 0,4 mmol de cyclohexénone. Le mélange réactionnel est chauffé à 100°C pendant 5 heures. Après retour à température ambiante, les solvants sont évaporés sous pression réduite. Le résidu obtenu est dissous dans 20 ml d'acétate d'éthyle, lavé avec 5 ml d'une solution aqueuse saturée en hydrogénocarbonate de sodium, puis séché sur sulfate de sodium. Les solvants sont ensuite évaporés sous pression réduite. Le produit est purifié par filtration sur silice. On isole ainsi la (S)-3-phénylcyclohexanone caractérisée par le spectre RMN¹H suivant.
RMN ¹H (200MHz) CDCl₃ : 1,84 (2H, m) ; 2,16 (2H, m) ; 2,46 (4H, m) ; 3,0 (1H, m) ; 7,21-7,45 (5H, m)
Excès énantiomérique : 96 % ee (déterminé par CPG chiral Lipodex A)

### EXEMPLE 8

Le TABLEAU 2 ci-après présente un comparatif des résultats d'hydrogénation de différents substrats obtenus, d'une part, avec les complexes de ruthénium selon l'invention et, d'autre part, avec les complexes de type Ru-Binap, dans les mêmes conditions opératoires (Température, Pression et Solvant).

Le **TABLEAU 2** présente une comparaison des résultats obtenus au **TABLEAU 1** pour les complexes selon l'invention et de ceux obtenus avec les complexes correspondants dans lequel le ligand (I) selon l'invention a été remplacé par le ligand BINAP.

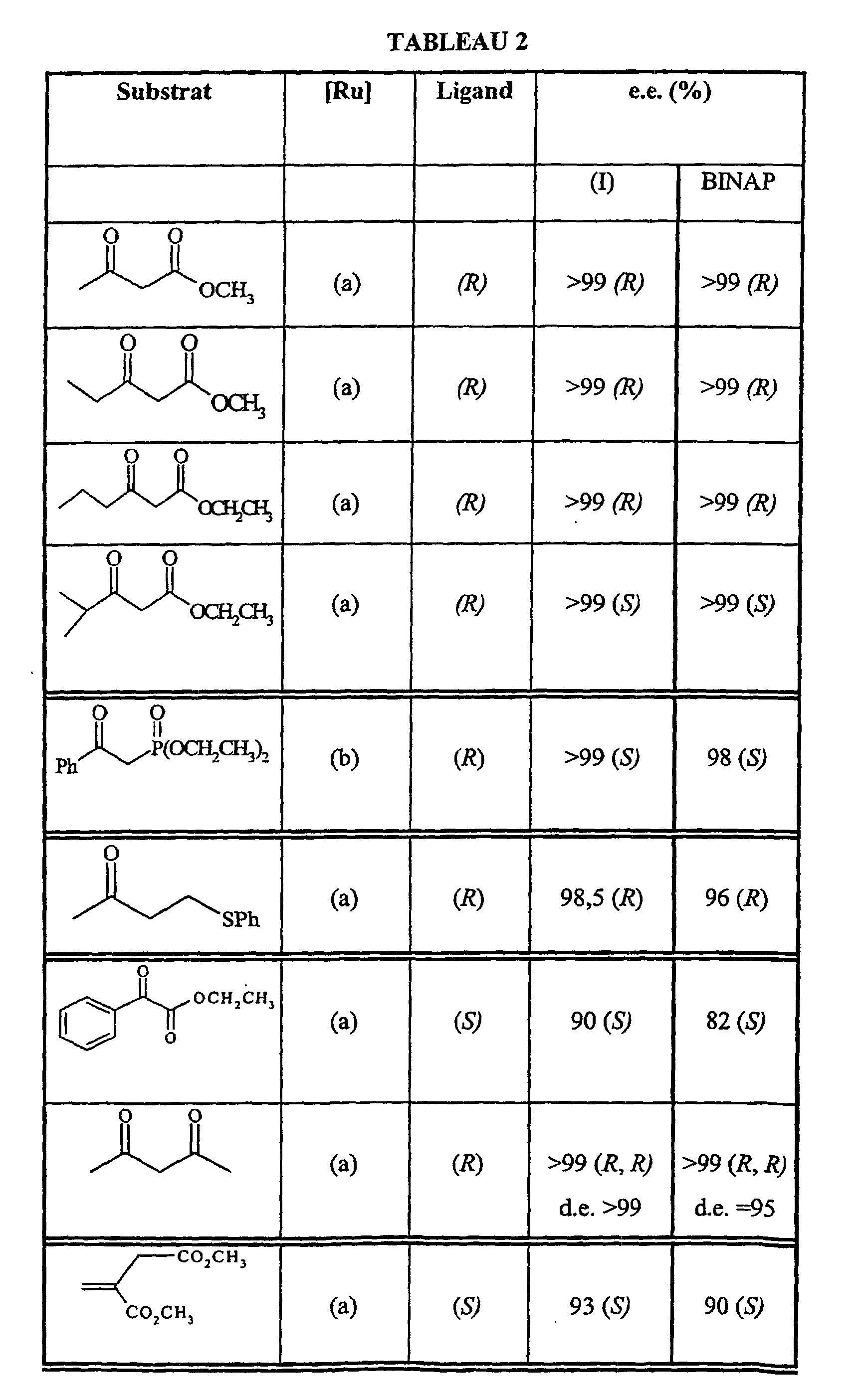

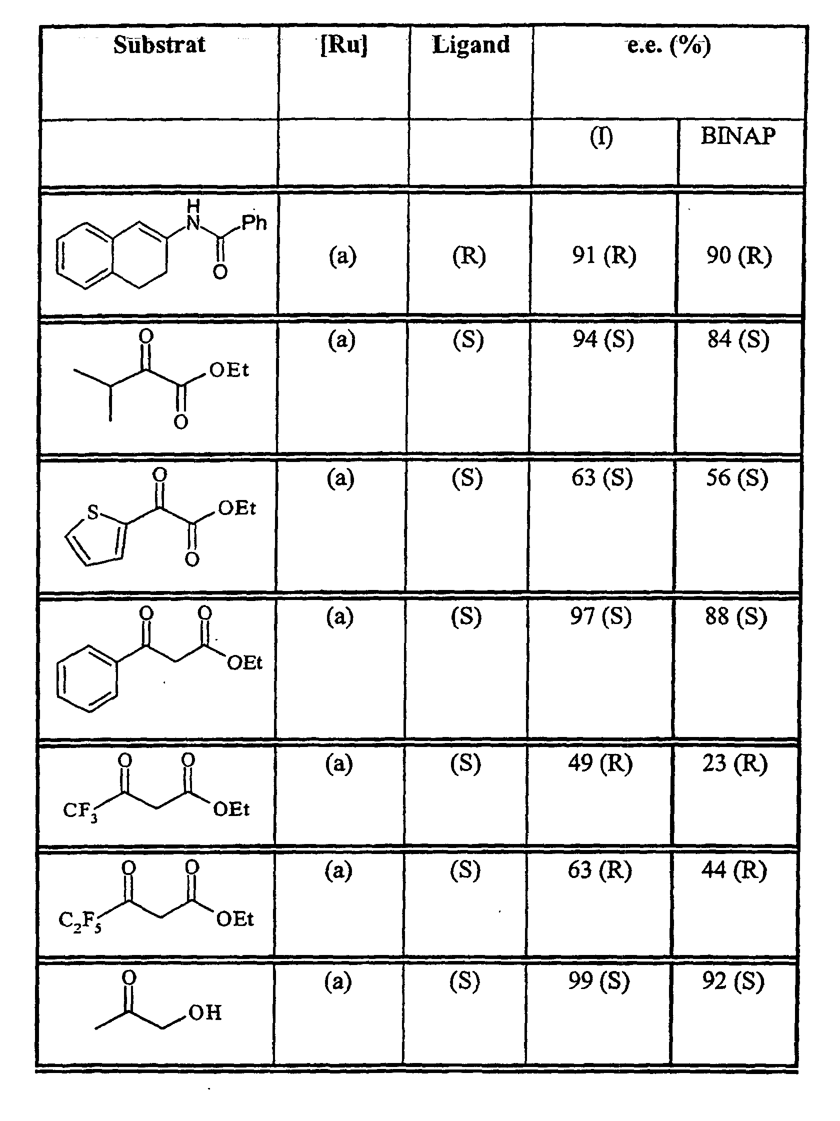

## Revendications

1. Composés de formule (I) sous forme optiquement pure ou racémique : 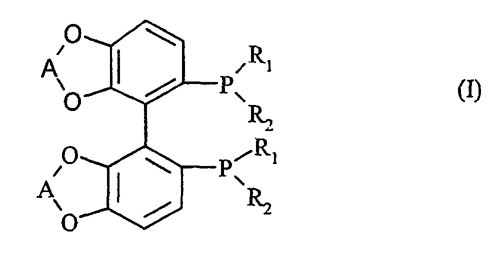 dans laquelle :
- R₁ et R₂ représentent chacun indépendamment :
- un groupe (C₅-C₇)cycloalkyle, un groupe phényle éventuellement substitué par un ou plusieurs groupe (C₁-C₄)alkyle, (C₁-C₄)alcoxy, di(C₁-C₄)alkylamino, ou par un atome d'halogène, ou
- un groupe hétéroaryle à 5 chaînons ;
- A représente un groupe éthylène (CH₂-CH₂) ou un groupe CF₂.

2. Composés de formule (I) selon la revendication 1, **caractérisés en ce que** R₁ et R₂ sont identiques.

3. Composés de formule (I) selon la revendication 2, **caractérisés en ce que** R₁ et R₂ représentent un groupe phényle.

4. Composés intermédiaires de formule (IIA) utiles pour la préparation de composés de formule (I) selon la revendication 1: 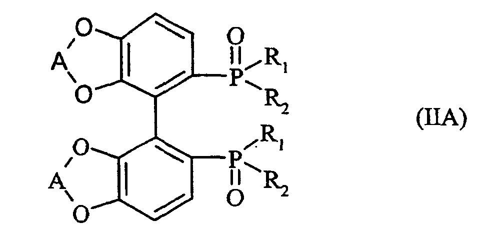 dans laquelle R₁, R₂ et A sont tels que définis pour (I) à la revendication 1.

5. Composés intermédiaires de formule (IIB) utiles pour la préparation de composés de formule (1) selon la revendication 1 : 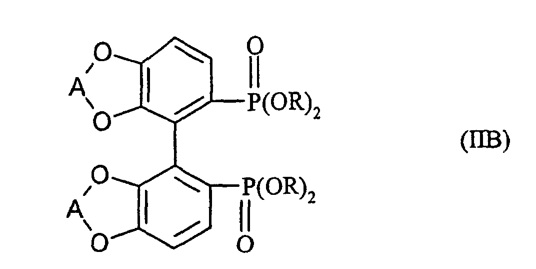 dans laquelle A est tel que défini pour (I) et R représente un groupe (C₁-C₄) alkyle ou un groupe phényle éventuellement substitué.

6. Composés intermédiaires de formule (IIIA) utiles pour la préparation de composés de formule (I) selon la revendication 1 : 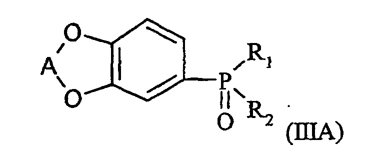 dans laquelle R₁, R₂ et A sont tels que définis pour (I) à la revendication 1.

7. Composés intermédiaires de formule (IIIC) utiles pour la préparation de composés de formule (I) selon la revendication 1 : 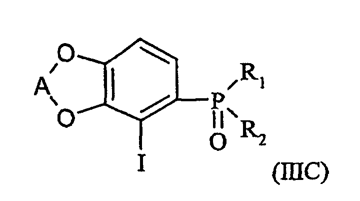 dans laquelle R₁, R₂ et A sont tels que définis pour (I) à la revendication 1.

8. Composés intermédiaires de formule (IVA) utiles pour la préparation de composés de formule (I) selon la revendication 1 : 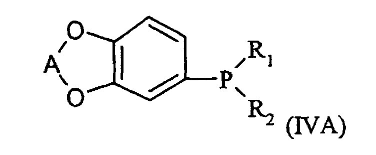 dans laquelle R₁, R₂ et A sont tels que définis pour (I) à la revendication 1.

9. Utilisation d'un composé de formule (I) selon l'une quelconque des revendications 1 à 3, comme ligand pour la préparation d'un complexe métallique, utile comme catalyseur chiral dans la catalyse asymétrique.

10. Catalyseur métallique chiral **caractérisé en ce qu'**il comprend au moins un ligand choisi parmi un composé de formule (I) selon l'une quelconque des revendications 1 à 3.

11. Catalyseurs métalliques chiraux selon la revendication 10, **caractérisés en ce que** le ligand choisi parmi un composé de formule (I) selon l'une quelconque des revendications 1 à 3, est sous forme optiquement pure.

12. Catalyseur métallique chiral selon la revendication 10 ou 11, **caractérisé en ce que** le métal est choisi parmi le rhodium, le ruthénium, l'iridium, le palladium, le cuivre ou le nickel.

13. Catalyseur métallique chiral selon la revendication 12 de formule (VI) :
MₘLₙXₚS_{q} (VI)
dans laquelle :
- M représente un métal choisi parmi le rhodium, le ruthénium, l'iridium, le palladium , le nickel ou le cuivre ;
- L représente un composé de formule (I) selon l'une des revendications 1 à 3 ;
- X, S, m, n, p et q sont définis comme suit :
• lorsque M = Rh, alors X = Cl, Br ou I ; m = n = p = 2 ; q = 0 ;
• lorsque M = Ru, alors :
X = -OC(O)CH₃; m = n = 1 ; p = 2 ; q = 0
ou bien X = Br ; m = n = 1 ; p = 2 ; q = 0 ;
ou bien X = Cl ; S = N(CH₂CH₃)₃ ; m = n = 2 ; p = 4 ; q = 1 ;
ou bien X = méthylallyl ; m = n =1 ; p = 2 ; q = 0 ;
ou bien X = Cl ; S = pyridine ; m = n = 1; p = q = 2;
ou bien X = Cl ; S = 1,2-diamine chirale ; m = n = 1 ; p = q = 2 ou p = 2, q = 1;
• lorsque M = Ir, alors X = Cl, Br ou I ; m = n = p = 2 ; q = 0 ;
• lorsque M = Pd, alors :
X = Cl ; m = n = 1 ; p = 2 ; q = 0;
ou bien X = π-allyl ; m = n = p = 2 ; q = 0;
• lorsque M = Ni, alors X = Cl, Br ou I; m = n = 1; p = 2; q = 0.

14. Catalyseur métallique chiral selon la revendication 12 de formule (VII) :
[Mᵣ Lₛ Zₜ Wᵤ]Yᵥ (VII)
dans laquelle :
- M représente un métal choisi parmi le rhodium, le ruthénium, l'iridium, ou le palladium ou le cuivre ;
- L représente un composé de formule (I) selon l'une des revendications 1 à 3 ;
- Z, W, r, s, t, u et v sont définis comme suit :
• lorsque M = Rh, alors Z = 1,5-cyclooctadiène ou norbornadiène ; Y = BF₄, ClO₄ PF₆, OTf ou BPh₄ ; r = s = t = v = 1 ; u = 0;
• lorsque M = Ru, alors :
Z = Cl, Br ou I ; W = benzène ou *p*-cymène ; Y = Cl, Brou I;r=s=t=u=v=1 ;
ou bien Y = BF₄, ClO₄, PF₆ ou BPh₄ ; r = s =1 ; t = u = 0 ; v = 2;
ou bien Z = Cl ; Y = NH₂(C₂H₅)₂ ; r = s = 2 ; t = 5 ; u = 0 ; v = 2;
• lorsque M = Ir, alors Z = 1,5-cyclooctadiène ou norbornadiène ; Y = BF₄, ClO₄, PF₆ ou BPh₄ ; r = s = v = 1 ; t = 1, u = 0;
• lorsque M = Pd, alors Y = BF₄, ClO₄, PF₆ ou BPh₄ ; r = s = v =1 ; t = u = 0 ;
• lorsque M = Cu, alors Y = PF₆ ou ClO₄ ; r = s = v = 1 ; t = u = 0.

15. Catalyseur de formule (VI) selon la revendication 13, **caractérisé en ce que** M = Ru et
• X = Br ; m = n = 1; p = 2; q = 0;
• ou X = Cl ; S = N(CH₂CH₃)₃ ; m = n = 1 ; p = 4 ; q = 1 ;
• ou X = Cl ; S = pyridine ; m = n = l ; p = q = 2.

16. Catalyseur de formule (VII) selon la revendication 14, **caractérisé en ce que** M = Rh, Z = 1,5-cyclooctadiène ou norbornadiène ; Y = BF₄, ClO₄, PF₆, bTf ou BPh₄; r = s = t = v = 1 ; et u = 0.

17. Procédé d'hydrogénation catalytique **caractérisé en ce qu'**il utilise un catalyseur selon l'une quelconque des revendications 10 à 16.

18. Utilisation d'un catalyseur métallique selon l'une quelconque des revendications 10 à 16, pour catalyser des réactions asymétriques, notamment d'hydrogénation et de formation de liaison C-C.

## Patentansprüche

1. Verbindungen der Formel (I) in optisch reiner oder racemischer Form: 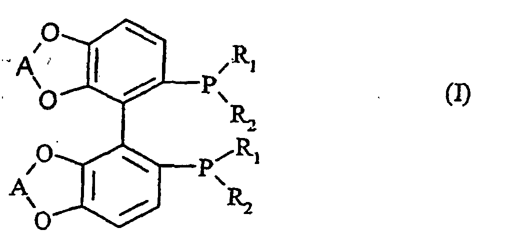 wobei:
R₁ und R₂ jeweils unabhängig
- einen (C₅-C₇)-Cycloalkylrest, eine Phenylgruppe, die gegebenenfalls mit einem oder mehreren (C₁-C₄)-Alkyl-, (C₁-C₄)-Alkoxy-, Di(C₁-C₄)-alkylaminorest oder mit einem Halogenatom substituiert ist, oder
- einen 5-gliedrigen Heteroarylrest
darstellen; und
A eine Ethylengruppe (CH₂-CH₂) oder eine Gruppe CF₂ darstellt.

2. Verbindungen der Formel (I) gemäß Anspruch 1, **dadurch gekennzeichnet, dass** R₁ und R₂ gleich sind.

3. Verbindungen der Formel (I) gemäß Anspruch 2, **dadurch gekennzeichnet, dass** R₁ und R₂ eine Phenylgruppe darstellen.

4. Zwischenverbindungen der Formel (IIA), die zur Herstellung von Verbindungen der Formel (I) gemäß Anspruch 1 verwendbar sind: 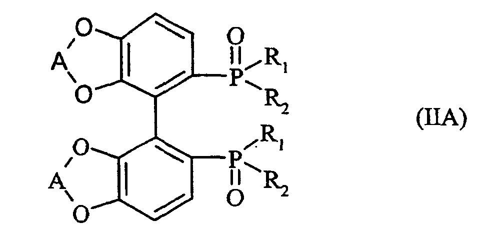 wobei R₁, R₂ und A wie für (I) in Anspruch 1 angegeben definiert sind.

5. Zwischenverbindungen der Formel (IIB), die zur Herstellung von Verbindungen der Formel (I) gemäß Anspruch 1 verwendbar sind: 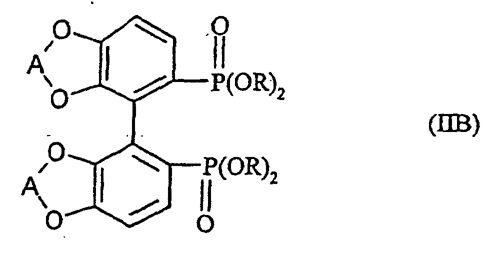 wobei A wie für (I) angegeben definiert ist und R einen (C₁-C₄)-Alkylrest oder eine gegebenenfalls substituierte Phenylgruppe darstellt.

6. Zwischenverbindungen der Formel (IIIA), die zur Herstellung von Verbindungen der Formel (I) gemäß Anspruch 1 verwendbar sind: 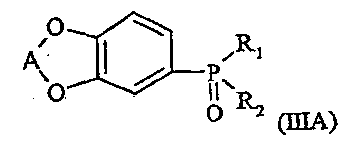 wobei R₁, R₂ und A wie für (I) in Anspruch 1 angegeben definiert sind.

7. Zwischenverbindungen der Formel (IIIC), die zur Herstellung von Verbindungen der Formel (I) gemäß Anspruch 1 verwendbar sind: 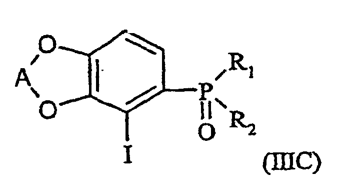 wobei R₁, R₂ und A wie für (I) in Anspruch 1 angegeben definiert sind.

8. Zwischenverbindungen der Formel (IVA), die zur Herstellung von Verbindungen der Formel (I) gemäß Anspruch 1 verwendbar sind: 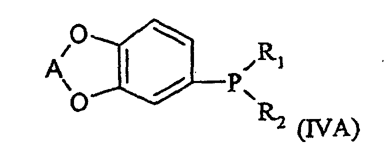 wobei R₁, R₂ und A wie für (I) in Anspruch 1 angegeben definiert sind.

9. Verwendung einer Verbindung der Formel (I) gemäß einem der Ansprüche 1 bis 3, als Ligand zur Herstellung eines Metallkomplexes, der als chiraler Katalysator in der asymmetrischen Katalyse verwendbar ist.

10. Chiraler Metallkatalysator, **dadurch gekennzeichnet, dass** er mindestens einen Liganden, der aus einer Verbindung der Formel (I) gemäß einem der Ansprüche 1 bis 3 ausgewählt ist, umfasst.

11. Chirale Metallkatalysatoren gemäß Anspruch 10, **dadurch gekennzeichnet, dass** der Ligand, der aus einer Verbindung der Formel (I) gemäß einem der Ansprüche 1 bis 3 ausgewählt ist, in optisch reiner Form vorliegt.

12. Chiraler Metallkatalysator gemäß Anspruch 10 oder 11, **dadurch gekennzeichnet, dass** das Metall aus Rhodium, Ruthenium, Iridium, Palladium, Kupfer oder Nickel ausgewählt ist.

13. Chiraler Metallkatalysator gemäß Anspruch 12 mit der Formel (VI):
MₘLₙXₚS_{q} (VI)
wobei:
- M ein Metall ausgewählt aus Rhodium, Ruthenium, Iridium, Palladium, Nickel oder Kupfer darstellt;
- L eine Verbindung der Formel (I) gemäß einem der Ansprüche 1 bis 3 darstellt; und
- X, S, m, n, p und q wie folgt definiert sind:
• wenn M = Rh, dann ist X = Cl, Br oder I; m = n = p = 2; q = 0;
• wenn M = Ru, dann ist:
• X = -OC(O)CH₃ ; m = n = 1 ; p = 2 ; q = 0;
oder X = Br; m = n = 1 ; p = 2 ; q = 0;
oder X = Cl; S = N(CH₂CH₃)₃; m = n = 2; p = 4; q = 1;
oder X = Methylallyl; m = n = 1; p = 2; q = 0;
oder X = Cl; S = Pyridin; m = n = 1; p = q = 2;
oder X = Cl; S = chirales 1,2-Diamin; m = n = 1; p = q = 2 oder p = 2, q = 1;
• wenn M = Ir, dann ist X = Cl, Br oder I; m = n = p = 2; q = 0;
• wenn M = Pd, dann ist:
X = Cl; m = n = 1; p = 2; q = 0;
oder X=π-Allyl; m = n = p = 2; q = 0;
• wenn M = Ni, dann ist X = Cl, Br oder I; m = n = 1; p = 2, q = 0.

14. Chiraler Metallkatalysator gemäß Anspruch 12 mit der Formel (VII):
[MᵣLₛZₜWᵤ]Yᵥ (VII)
wobei:
- M ein Metall, ausgewählt aus Rhodium, Ruthenium, Iridium, Palladium oder Kupfer, darstellt:
- L eine Verbindung der Formel (I) gemäß einem der Ansprüche 1 bis 3 darstellt; und
- Z, W, r, s, t, u und v wie folgt definiert sind:
• wenn M = Rh, dann ist Z = 1,5-Cyclooctadien oder Norbomadien; Y = BF₄, ClO₄, PF₆,OTf oder BPh₄; r = s = t = v = 1; u = 0;
• wenn M = Ru, dann ist:
Z = Cl, Br oder I; W = Benzol oder p-Cymol; Y = Cl, Br oder I; r = s = t = u = v = 1;
oder Y = BF₄, ClO₄, PF₆ oder BPh₄; r = s = 1; t = u = 0; v = 2;
oder Z = Cl; Y = NH₂(C₂H₅)₂ ; r = s = 2; t = 5; u = 0; v = 1;
• wenn M = Ir, dann ist Z = 1,5-Cycloctadien und Norbomadien; Y = BF₄, ClO₄, PF₆ oder BPh₄; r = s = v = 1; t = 1; u = 0;
• wenn M = Pd, dann ist Y = BF₄, ClO₄, PF₆ oder BPh₄; r = s = v = 1; t = u = 0;
• wenn M = Cu, dann ist Y = PF₆ oder ClO₄ ; r = s = v = 1; t = u = 0.

15. Katalysator der Formel (VI) gemäß Anspruch 13, **dadurch gekennzeichnet, dass** M = Ru ist und
X = Br; m = n = 1; p = 2; q = 0;
oder X = Cl; S = N(CH₂CH₃)₃; m = n = 1; p= 4; q = 1;
oder X = Cl; S = Pyridin; m = n = 1; p = q = 2 ist.

16. Katalysator der Formel (VII) gemäß Anspruch 14, **dadurch gekennzeichnet, dass** M = Rh, Z = 1,5-Cyclooctadien oder Norbomadien; Y = BF₄, ClO₄, PF₆, OTf oder BPh₄; r = s = t = v = 1; und u = 0 ist.

17. Katalytisches Hydrierverfahren, **dadurch gekennzeichnet, dass** es einen Katalysator gemäß einem der Ansprüche 10 bis 16 verwendet.

18. Verwendung eines Metallkatalysators gemäß einem der Ansprüche 10 bis 16 zur Katalyse von asymmetrischen Reaktionen, insbesondere der Hydrierung und der Bildung von C-C-Bindungen.

## Claims

1. Compounds, in optically pure or racemic form, of formula (I): 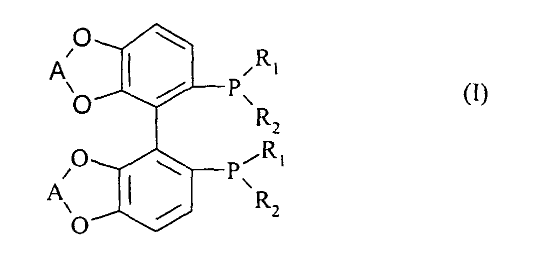 in which:
- R₁ and R₂ each independently are:
- a (C₅-C₇)cycloalkyl group, a phenyl group optionally substituted by one or more (C₁-C₄)alkyl, (C₁-C₄)alkoxy or di(C₁-C₄)alkylamino groups or by a halogen atom, or
- a 5-membered heteroaryl group; and
- A is an ethylene group (CH₂-CH₂) or a CF₂ group.

2. Compounds of formula (I) according to claim 1, **characterized in that** R₁ and R₂ are identical.

3. Compounds of formula (I) according to claim 2, **characterized in that** R₁ and R₂ are a phenyl group.

4. Intermediate compounds of formula (IIA) useful for the preparation of compounds of formula (I) according to claim 1: 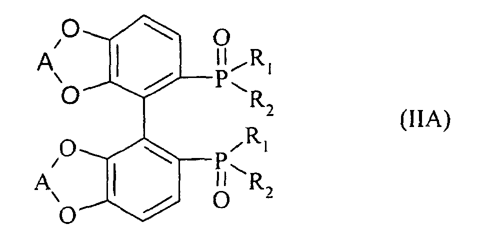 in which R₁, R₂ and A are as defined for (I) in claim 1.

5. Intermediate compounds of formula (IIB) useful for the preparation of compounds of formula (I) according to claim 1: 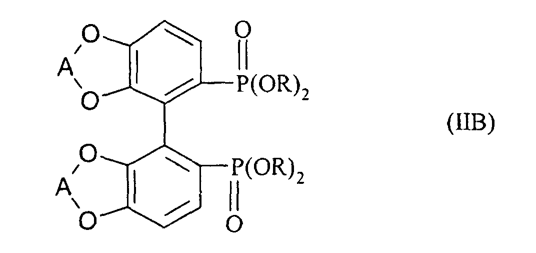 in which A is as defined for (I) and R is a (C₁-C₄)alkyl group or an optionally substituted phenyl group.

6. Intermediate compounds of formula (IIIA) useful for the preparation of compounds of formula (I) according to claim 1: 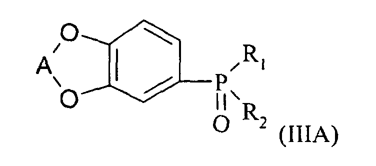 in which R₁, R₂ and A are as defined for (I) in claim 1.

7. Intermediate compounds of formula (IIIC) useful for the preparation of compounds of formula (I) according to claim 1: 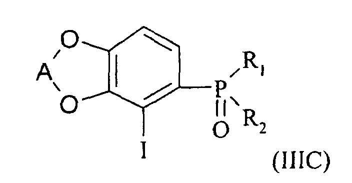 in which R₁, R₂ and A are as defined for (I) in claim 1.

8. Intermediate compounds of formula (IVA) useful for the preparation of compounds of formula (I) according to claim 1: 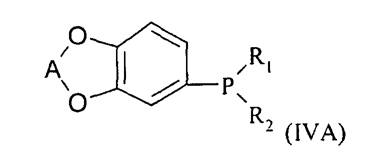 in which R₁, R₂ and A are as defined for (I) in claim 1.

9. Use of a compound of formula (I) according to any one of claims 1 to 3 as a ligand for the preparation of a metal complex useful as a chiral catalyst in asymmetric catalysis.

10. Chiral metal catalyst, **characterized in that** it comprises at least one ligand selected from a compound of formula (I) according to any one of claims 1 to 3.

11. Chiral metal catalysts according to claim 10, **characterized in that** the ligand selected from a compound of formula (I) according to any one of claims 1 to 3 is in optically pure form.

12. Chiral metal catalyst according to claim 10 or 11, **characterized in that** the metal is selected from rhodium, ruthenium, iridium, palladium, copper and nickel.

13. Chiral metal catalyst according to claim 12 of formula (VI):
MₘLₙXₚS_{q} (VI)
in which:
- M is a metal selected from rhodium, ruthenium, iridium, palladium, nickel and copper;
- L is a compound of formula (I) according to one of claims 1 to 3; and
- X, S, m, n, p and q are defined as follows:
• if M = Rh, then X = Cl, Br or I; m = n = p = 2; q = 0;
• if M = Ru, then: X = -OC(O)CH₃; m = n = 1; p = 2; q = 0;
or X = Br; m = n = 1; p = 2; q = 0;
or X = Cl; S = N(CH₂CH₃)₃; m = n = 2; p = 4; q = 1;
or X = methylallyl; m = n = 1; p = 2; q = 0;
or X = Cl; S = pyridine; m = n = 1; p = q = 2;
or X = Cl; S = chiral 1,2-diamine; m = n = 1; p = q = 2 or p = 2, q = 1;
• if M = Ir, then X = C), Br or I; m = n = p = 2; q = 0;
• if M = Pd, then: X = Cl; m = n = 1; p = 2; q = 0;
or X = π-allyl; m = n = p = 2; q = 0;
• if M = Ni, then X = Cl, Br or I; m = n = 1; p = 2; q = 0.

14. Chiral metal catalyst according to claim 12 of formula (VII):
[Mᵣ-LₛZₜWᵤ]Yᵥ (VII)
in which:
- M is a metal selected from rhodium, ruthenium, iridium, palladium and copper;
- L is a compound of formula (1) according to one of claims 1 to 3; and
- Z, W, r, s, t, u and v are defined as follows:
• if M = Rh, then Z = 1,5-cyclooctadiene or norbornadiene; Y = BF₄, ClO₄, PF₆, OTf or BPh₄; r = s = t = v = 1; u = 0;
• if M = Ru, then: Z = Cl, Br or I; W = benzene or *p*-cymene; Y = Cl, Br or I;
r = s = t = u = v = 1;
or Y = BF₄, ClO₄, PF₆ or BPh₄; r = s = 1; t = u = 0; v = 2;
or Z = Cl; Y = NH₂(C₂H₅)₂; r = s = 2; t = 5; u = 0; v = 1;
• if M = Ir, then Z = 1,5-cyclooctadiene or norbomadiene; Y = BF₄, ClO₄, PF₆ or BPh₄; r = s = v = 1; t = 1; u = 0;
• if M = Pd, then Y = BF₄, ClO₄, PF₆ or BPh₄; r = s = v = 1; t = u = 0;
• if M = Cu, then Y = PF₆ or ClO₄; r = s = v = 1; t = u = 0.

15. Catalyst of formula (VI) according to claim 13, **characterized in that** M = Ru and
• X = Br; m = n = 1; p = 2; q = 0;
• or X = Cl; S = N(CH₂CH₃)₃; m = n =: 1; p = 4; q = 1;
• or X = Cl;S = pyridine; m = n = 1; p = q = 2.

16. Catalyst of formula (VII) according to claim 14, **characterized in that** M = Rh and Z = 1,5-cyclooctadiene or norbomadiene; Y = BF₄, ClO₄, PF₆, OTf or BPh₄; r = s = t = v = 1; u = 0.

17. Catalytic hydrogenation process, **characterized in that** it uses a catalyst according to any one of claims 10 to 16.

18. Use of a metal catalyst according to any one of claims 10 to 16 for the catalysis of asymmetric reactions, especially hydrogenation reactions and reactions for the formation of C-C bonds.
